(19) Europäisches Patentamt
European Patent Office
Office européen des brevets

(11) Veröffentlichungsnummer: **0 173 320**
**A1**

(12) # EUROPÄISCHE PATENTANMELDUNG

(21) Anmeldenummer: **85110832.4**

(22) Anmeldetag: **19.08.85**

(51) Int. Cl.⁴: **C 07 D 239/42**, C 07 D 417/12, A 01 N 47/44

(30) Priorität: **30.08.84 DE 3431920**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-5000 Köln 80 (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

(54) **Substituierte Carbonylphenylsulfonylguanidine.**

(57) Die Erfindung betrifft neue substituierte Carbonylphenylsulfonylguanidine der allgemeinen Formel (I)

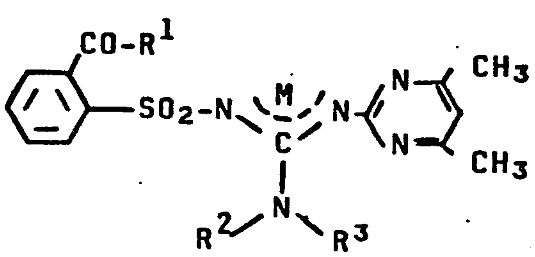

(I)

(worin die Reste $R^1$, $R^2$, $R^3$ und M die in der Beschreibung angegebenen Bedeutungen haben), sowie 1:1-Addukte von Verbindungen der Formel (I) mit starken Säuren, mehrere Verfahren und neue Zwischenprodukte zu ihrer Herstellung und ihre Verwendung als Herbizide.

EP 0 173 320 A1

0 173 320

BAYER AKTIENGESELLSCHAFT
5090 Leverkusen,Bayerwerk

Konzernverwaltung RP
Patentabteilung

Bi/mü
Ib

## Substituierte Carbonylphenylsulfonylguanidine

Die Erfindung betrifft neue Carbonylphenylsulfonylguanidine, Verfahren und neue Zwischenprodukte zu ihrer Herstellung und ihre Verwendung als Herbizide.

Verschiedene Guanidin-Derivate sind aus Patentschriften (vgl. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt. Weitere Guanidin-Derivate sind Gegenstand einer nicht zum vorveröffentlichten Stand der Technik gehörenden Patentanmeldung der Anmelderin (vgl. DE-OS 3 334 455).

Es wurden nun neue substituierte Carbonylphenylsulfonylguanidine der allgemeinen Formel (I)

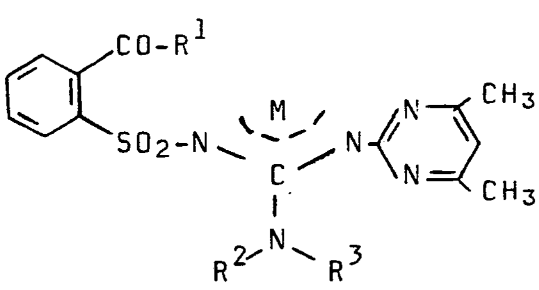

(I)

in welcher

M für Wasserstoff oder ein Metalläquivalent steht,

$R^1$ für Hydroxy, $C_3$-$C_8$-Alkoxy und - für den Fall, daß $R^2$ von Wasserstoff verschieden ist - auch für Ethoxy steht und weiter für $C_3$-$C_6$-Cycloalkoxy, $C_3$-$C_6$-Cycloalkyl-$C_1$-$C_2$-alkoxy, $C_1$-$C_6$-Halogenalkoxy, Cyano-$C_1$-$C_6$-alkoxy, $C_1$-$C_4$-Alkoxy-$C_1$-$C_4$-alkoxy, $C_1$-$C_4$-Alkylthio-$C_1$-$C_4$-alkoxy, $C_1$-$C_4$-Alkylsulfinyl-$C_1$-$C_4$-alkoxy, $C_1$-$C_4$-Alkylsulfonyl-$C_1$-$C_4$-alkoxy, $C_1$-$C_4$-Alkoxy-carbonyl-$C_1$-$C_2$-alkoxy, $C_3$-$C_8$-Alkenoxy, $C_3$-$C_8$-Alkinoxy, Phenyl-$C_1$-$C_2$-alkoxy, Phenoxy [welches gegebenenfalls durch Halogen,Cyano, Nitro, $C_1$-$C_4$-Alkyl, $C_1$-$C_2$-Halogenalkyl, $C_1$-$C_4$-Alkoxy, $C_1$-$C_2$-Halogenalkoxy oder $C_1$-$C_4$-Alkoxycarbonyl substituiert ist], für $C_1$-$C_8$-Alkylamino [welches gegebenenfalls durch Halogen, Hydroxy,Cyano, $C_1$-$C_4$-Alkoxy oder $C_1$-$C_4$-Alkoxy-carbonyl substituiert ist], $C_3$-$C_6$-Cycloalkylamino, $C_3$-$C_6$-Cyclo-alkyl-$C_1$-$C_2$-alkyl-amino,$C_3$-$C_8$-Alkenylamino, $C_3$-$C_8$-Alkinylamino, Phenyl-$C_1$-$C_3$-alkylamino, für Di-($C_1$-$C_4$-alkyl)-amino [welches gegebenenfalls durch Halogen,Cyano Hydroxy oder $C_1$-$C_4$-Alkoxy substituiert ist], Di-($C_3$-$C_6$-Cycloalkyl)-amino,Di-($C_3$-$C_5$-alkenyl)-amino, Di-($C_3$-$C_5$-alkinyl)-amino, für $C_1$-$C_8$-Alkoxyamino [welches gegebenenfalls durch Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_8$-Alkenyloxyamino, $C_3$-$C_8$-Alkinyloxyamino, $C_3$-$C_6$-Cyclo-alkyloxyamino, $C_1$-$C_4$-Alkoxy-carbonyl-$C_1$-$C_2$-alkoxy-

amino, Phenyl-$C_1$-$C_2$-alkoxyamino [welches gegebenenfalls durch Halogen, Cyano, Nitro, $C_1$-$C_4$-Alkyl, $C_1$-$C_4$-Alkoxy oder $C_1$-$C_4$-Alkoxy-carbonyl substituiert ist], für $C_1$-$C_6$-Alkylhydrazino, Di-($C_1$-$C_4$-alkyl)-hydrazino, $C_3$-$C_6$-Cycloalkylhydrazino, Phenyl-$C_1$-$C_2$-alkylhydrazino, Phenylhydrazino [welches gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-Alkoxy-carbonyl substituiert ist], für $C_1$-$C_4$-Alkyl-carbonyl-hydrazino, $C_1$-$C_4$-Alkoxy-carbonyl-hydrazino, $C_1$-$C_4$-Alkyl-sulfonylhydrazino, $C_1$-$C_4$-Halogenalkylsulfonylhydrazino oder Phenylsulfonylhydrazino [welches gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy oder $C_1$-$C_4$-Alkoxy-carbonyl substituiert ist] oder für Amino steht;

in welcher weiter

$R^2$ für Wasserstoff, $C_1$-$C_6$-Alkyl [welches gegebenenfalls durch Fluor, Chlor, Brom Cyano, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für den Rest

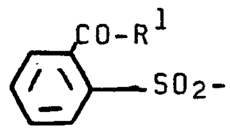 steht, worin

$R^1$ die oben angegebene Bedeutung hat;

in welcher weiter

$R^3$ für Wasserstoff, $C_1$-$C_6$-Alkyl [welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht, oder

$R^2$ und $R^3$ gemeinsam für $C_4$-$C_6$-Alkandiyl stehen, [welches gegebenenfalls durch eine Sauerstoff-Brücke oder durch eine Brücke >N-$R^4$ unterbrochen ist], wobei

$R^4$ für $C_1$-$C_4$-Alkyl, $C_1$-$C_4$-Alkyl-carbonyl, $C_1$-$C_4$-Alkoxy-carbonyl oder Phenyl steht [welches gegebenenfalls durch Fluor, Chlor, Brom, Cyano, Nitro, $C_1$-$C_4$-Alkyl, $C_1$-$C_2$-Fluoralkyl oder $C_1$-$C_4$-Alkoxy substituiert ist];

in welcher weiter

$R^3$ für den Rest $-O-R^5$ steht, worin

$R^5$ für $C_1$-$C_8$-Alkyl [welches gegebenenfalls durch Halogen, $C_1$-$C_4$-Alkoxy, $C_1$-$C_4$-Alkylthio, $C_1$-$C_4$-Alkylsulfonyl substituiert ist] $C_3$-$C_6$-Alkenyl /welches gegebenenfalls durch Fluor oder Chlor substituiert ist/, $C_3$-$C_6$-Alkinyl-, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonylmethyl, $C_1$-$C_4$-Alkylamino-carbonyl-methyl, Di-($C_1$-$C_4$-alkyl)-amino-carbonyl-methyl, Phenyl, Benzyl oder Phenylethyl [welche gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy oder $C_1$-$C_4$-Alkoxycarbonyl substituiert sind] steht;

in welcher weiter

$R^3$ für den Rest $-N\begin{matrix}R^6\\R^7\end{matrix}$ steht, worin

$R^6$ für Wasserstoff oder $C_1$-$C_4$-Alkyl steht und

$R^7$ für $C_1$-$C_4$-Alkyl [welches gegebenenfalls durch Halogen, Cyano, Nitro, $C_1$-$C_4$-Alkoxy oder $C_1$-$C_4$-Alkoxy-carbonyl substituiert ist], $C_3$-$C_6$-Cycloalkyl, Phenyl-$C_1$-$C_2$-alkyl, Phenyl [welches gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-

Alkoxy-carbonyl substituiert ist], für $C_1$-$C_4$-Alkyl-carbonyl, $C_1$-$C_4$-Alkoxy-carbonyl, $C_1$-$C_4$-Alkylsulfonyl, $C_1$-$C_4$-Halogenalkylsulfonyl oder Phenylsulfonyl [welches gegebenenfalls durch Halogen, Cyano,Nitro, $C_1$-$C_4$-Alkyl, $C_1$-$C_4$-Halogenalkyl, $C_1$-$C_4$-Alkoxy, $C_1$-$C_2$-Halogenalkoxy oder $C_1$-$C_4$-Alkoxy-carbonyl substituiert ist] steht,

sowie 1:1-Addukte von Verbindungen der Formel (I) mit starken Säuren gefunden.

0 173 320



Die allgemeine Formel (I) steht - wenn M für Wasserstoff steht - für die einzelnen Tautomeren der Formeln (IA) und (IB)

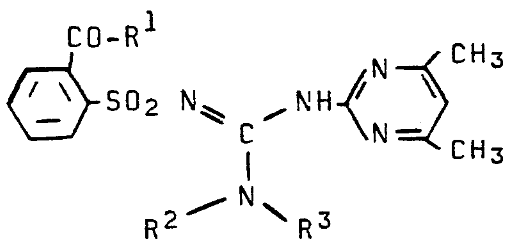

(IA)

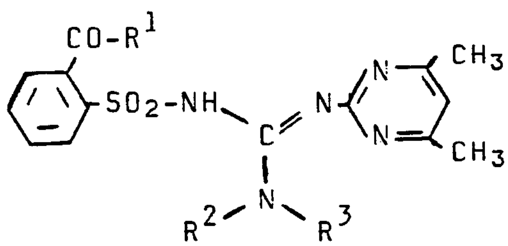

(IB)

in welchen

$R^1$, $R^2$ und $R^3$ die oben angegebenen Bedeutungen haben,

sowie für Gemische der Tautomeren (IA) und (IB).

Das Mischungsverhältnis hängt von aggregationsbestimmenden Faktoren, wie z.B. Temperatur, Lösungsmittel und Konzentration ab.

Für den Fall, daß neben M auch $R^2$ für Wasserstoff steht, ist eine weitere tautomere Form (IC) möglich:

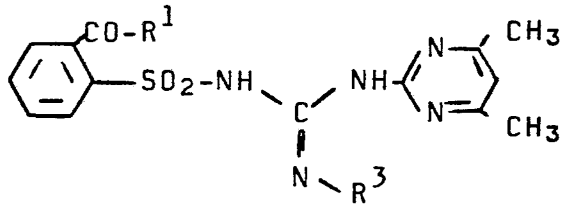

(IC)

Man erhält die neuen substituierten Carbonylphenylsulfonyl-guanidine der Formel (I)

(a) für den Fall, daß M für Wasserstoff steht und $R^2$ für den Rest 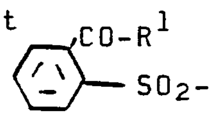 steht,

wenn man Guanidin-Derivate derFormel (II)

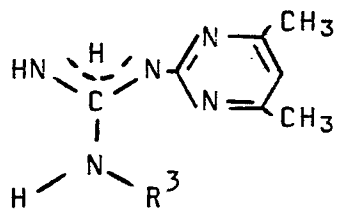

(II)

in welcher

$R^3$ die oben angegebene Bedeutung hat,

mit wenigstens zwei Moläquivalenten von substituierten Arensulfonsäurechloriden der Formel (III)

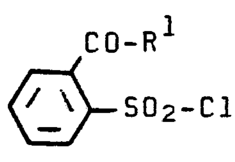

(III)

in welcher

$R^1$ die oben angegebene Bedeutung hat,

in Gegenwart von Säureakzeptoren und gegebenenfalls in Gegenwart von Verdünnungsmitteln umsetzt;

oder

(b) für den Fall, daß M für Wasserstoff steht und $R^2$ für Wasserstoff, $C_1$-$C_6$-Alkyl [welches gegebenenfalls wie oben angegeben substituiert ist], $C_3$-$C_6$-Cycloalkyl, $C_3$-$C_6$-Alkenyl, $C_3$-$C_6$-Alkinyl oder Benzyl [welches gegebenenfalls wie oben angegeben substituiert ist] steht oder zusammen mit $R^3$ die oben angegebene Bedeutung hat,

wenn man die nach dem unter (a) angegebenen Verfahren erhältlichen substituierten Carbonylphenylsulfonylguanidine der Formel (ID)

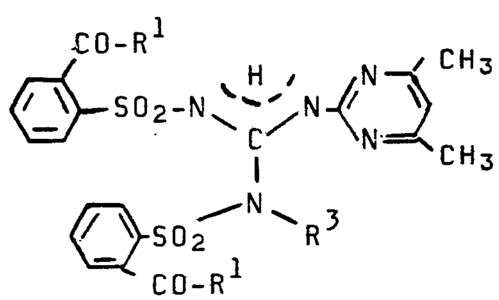

(ID)

in welcher

$R^1$ und $R^3$ die oben angegebenen Bedeutungen haben,

mit Aminoverbindungen der Formel (IV)

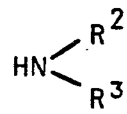 (IV)

in welcher

$R^2$ die vorausgehend unter (b) angegebene Bedeutung hat und

$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder

(c) für den Fall, daß M und $R^2$ für Wasserstoff stehen, wenn man Verbindungen der Formel (V)

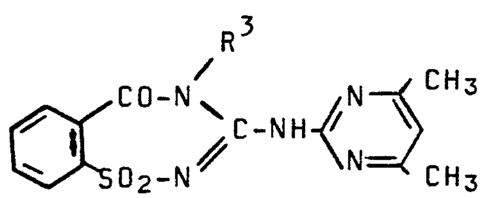 (V)

in welcher

$R^3$ die oben angegebene Bedeutung hat

mit Verbindungen der Formel (VI)

$M-R^1$ (VI)

in welcher

M und $R^1$ die oben angegebenen Bedeutungen haben,

gegebenenfalls in Gegenwart von Verdünnungsmitteln umsetzt;

oder

(d) für den Fall, daß M für ein Metalläquivalent steht, wenn man die nach den oben unter (a), (b) und (c) angegebenen Verfahren erhältlichen Verbindungen der Formel (I) in welcher

M für Wasserstoff steht und

$R^1$, $R^2$ und $R^3$ die oben angegebenen Bedeutungen haben,

mit Metallhydroxiden, -hydriden oder -alkanolaten oder mit metallorganischen Verbindungen gegebenenfalls in Gegenwart von Verdünnungsmitteln umsetzt;

oder

(e) für den Fall, daß 1:1-Addukte von Verbindungen der Formel (I) mit starken Säuren herzustellen sind,

wenn man Verbindungen der Formel (I), in welcher

M für Wasserstoff steht und

$R^1$, $R^2$ und $R^3$ die oben angegebenen Bedeutungen haben,

mit starken Säuren gegebenenfalls in Gegenwart von Verdünnungsmitteln umsetzt.

Die neuen substituierten Carbonylphenylsulfonylguanidine der Formel (I) und ihre Addukte mit starken Säuren zeichnen sich durch starke herbizide Wirksamkeit aus.

Ueberraschenderweise zeigen die neuen Verbindungen der Formel (I) erheblich bessere herbizide Wirkung als vorbekannte Guanidine gleicher Wirkungsrichtung.

Gegenstand der Erfindung sind vorzugsweise Verbindungen der Formel (I), in welcher

M für Wasserstoff oder ein Natrium-, Kalium- oder Calcium-äquivalent steht,

$R^1$ für $C_3$-$C_6$-Alkoxy und - für den Fall, daß $R^2$ von Wasserstoff verschieden ist - auch für Ethoxy, für $C_1$-$C_4$-Fluoralkoxy, $C_1$-$C_4$-Chloralkoxy, Cyano-$C_1$-$C_4$-alkoxy, $C_1$-$C_2$-Alkoxy-$C_1$-$C_2$-alkoxy, $C_1$-$C_2$-Alkoxy-carbonyl-$C_1$-$C_2$-alkoxy, $C_3$-$C_5$-Alkenoxy, $C_3$-$C_5$-Alkinoxy, Benzyloxy, Phenoxy [welches gegebenenfalls durch Fluor, Chlor, Brom, Cyano, Nitro, Methyl, Trifluormethyl, Methoxy, Trifluormethoxy oder Methoxycarbonyl substituiert ist], für $C_1$-$C_6$-Alkylamino [welches gegebenenfalls durch Fluor, Chlor, Hydroxy, Cyano, $C_1$-$C_2$-Alkoxy oder $C_1$-$C_2$-Alkoxy-carbonyl substituiert ist], $C_3$-$C_5$-Alkenylamino, $C_3$-$C_5$-Alkinylamino, Benzylamino, Phenylamino,

für Di-($C_1$-$C_3$-alkyl)-amino [welches gegebenenfalls durch Fluor, Chlor, Cyano, Hydroxy oder $C_1$-$C_2$-Alkoxy substituiert ist], für $C_1$-$C_6$-Alkoxyamino [welches gegebenenfalls durch Fluor, Chlor oder $C_1$-$C_2$-Alkoxy substituiert ist], $C_3$-$C_5$-Alkenoxyamino, $C_1$-$C_2$-Alkoxy-carbonyl-$C_1$-$C_2$-alkylamino, $C_1$-$C_2$-Alkoxy-carbonyl-$C_1$-$C_2$-alkoxyamino, Phenyl-$C_1$-$C_2$-alkoxyamino [welches gegebenenfalls durch Fluor, Chlor, Cyano, Nitro,Methyl, Methoxy oder Methoxycarbonyl substituiert ist], für $C_1$-$C_4$-Alkylhydrazino, Di-($C_1$-$C_2$-alkyl)-hydrazino, $C_3$-$C_6$-Cycloalkylhydrazino oder Phenylhydrazino [welches gegebenenfalls durch Fluor, Chlor, Cyano, Nitro, Methyl, Methoxy oder Methoxycarbonyl substituiert ist] , für $C_3$-$C_6$-Cycloalkoxy oder Amino steht;

in welcher weiter

$R^2$ für Wasserstoff, $C_1$-$C_4$-Alkyl [welches gegebenenfalls durch Fluor, Chlor, Brom, Cyano, Hydroxy oder $C_1$-$C_2$-Alkoxy substituiert ist], $C_3$-$C_6$-Cycloalkyl, $C_3$-$C_5$-Alkenyl, $C_3$-$C_5$-Alkinyl, Benzyl

oder den Rest 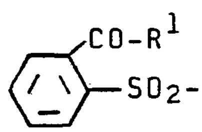 steht, worin

$R^1$ die oben angegebene Bedeutung hat,

in welcher weiter

$R^3$ für Wasserstoff, $C_1$-$C_4$-Alkyl [welches gegebenenfalls durch Fluor, Chlor, Brom, Cyano, Carboxy, $C_1$-$C_3$-Alkoxy-carbonyl, Hydroxy oder $C_1$-$C_2$-Alk-

oxy substituiert ist], $C_3$-$C_6$-Cycloalkyl, $C_3$-$C_5$-Alkenyl, $C_3$-$C_5$-Alkinyl, Phenylethyl, Benzyl [welches gegebenenfalls durch Fluor, Chlor, Nitro, Cyano, Methyl, Methoxy oder Methoxycarbonyl substituiert ist] oder Phenyl [ welches gegebenenfalls durch Fluor, Chlor, Brom, Hydroxy, Cyano, Nitro, Amino, $C_1$-$C_3$-Alkyl, Trifluormethyl, $C_1$-$C_3$-Alkoxy, Trifluormethoxy, $C_1$-$C_3$-Alkylthio, Trifluormethylthio, Aminosulfonyl oder $C_1$-$C_2$-Alkoxy-carbonyl substituiert ist] steht, oder

$R^2$ und $R^3$ gemeinsam für $C_4$-$C_5$-Alkandiyl stehen, welches gegebenenfalls durch eine Sauerstoff-Brücke oder durch eine Brücke >N-$R^4$ unterbrochen ist, wobei

$R^4$ für $C_1$-$C_3$-Alkyl, $C_1$-$C_3$-Alkyl-carbonyl oder Phenyl steht [welches gegebenenfalls durch Fluor, Chlor, Brom, Cyano, Nitro, $C_1$-$C_2$-Alkyl, Trifluormethyl oder $C_1$-$C_2$-Alkoxy substituiert ist];

in welcher weiter

$R^3$ für den Rest -O-$R^5$ steht, worin

$R^5$ für $C_1$-$C_8$-Alkyl [welches gegebenenfalls durch Fluor oder Chlor substituiert ist], $C_3$-$C_6$-Alkenyl, [welches gegebenenfalls durch Fluor oder Chlor substituiert ist],
$C_1$-$C_3$-Alkoxy-carbonyl-$C_1$-$C_2$-alkyl, Phenyl,

Phenylethyl oder Benzyl [welches gegebenenfalls

durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist] steht;

in welcher weiter

$R^3$ für den Rest 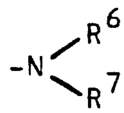 steht, worin

$R^6$ für Wasserstoff oder Methyl steht und

$R^7$ für $C_1$-$C_2$-Alkyl [welches gegebenenfalls durch Fluor, Chlor, Cyano, $C_1$-$C_2$-Alkoxy oder $C_1$-$C_2$-Alkoxy-carbonyl substituiert ist], $C_3$-$C_6$-Cycloalkyl, Phenylethyl, Benzyl oder Phenyl [welches gegebenenfalls durch Fluor, Chlor, Brom, Nitro, Cyano, $C_1$-$C_4$-Alkyl, $C_1$-$C_4$-Alkoxy oder $C_1$-$C_2$-Alkoxy-carbonyl substituliert ist] steht.

Gegenstand der Erfindung sind vorzugsweise ferner 1:1-Addukte von Verbindungen der Formel (I) - wie vorausgehend definiert, wobei M für Wasserstoff steht - mit Halogenwasserstoffsäuren, wie Hydrogenchlorid, Hydrogenbromid und Hydrogeniodid, mit Schwefelsäure, Trifluoressigsäure, mit gegebenenfalls durch Fluor oder Chlor substituierten Alkansulfonsäuren mit bis zu 4 Kohlenstoffatomen oder auch mit Benzol- oder Naphthalin-sulfonsäuren, welche gegebenenfalls durch Fluor, Chlor, Brom oder Methyl substituiert sind.

Gegenstand der Erfindung sind insbesondere Verbindungen der Formel (I), in welcher

(A) M für Wasserstoff oder ein Natrium-, Kalium- oder Calcium-äquivalent steht,

$R^1$ für $C_3$-$C_4$-Alkoxy, $C_1$-$C_3$-Fluoralkoxy, $C_1$-$C_3$-Chloralkoxy, Cyanoethoxy, Methoxyethoxy, $C_1$-$C_2$-Alkoxy-carbonyl-$C_1$-$C_2$-alkoxy, $C_3$-$C_4$-Alkenoxy, Benzyloxy oder Phenyloxy steht,

$R^2$ für den Rest 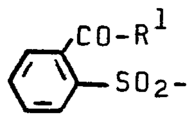 steht,worin

$R^1$ die vorausgehend angegebene Bedeutung hat, und

$R^3$ für den Rest $-O-R^5$ steht, worin

$R^5$ für $C_1$-$C_4$-Alkyl [welches gegebenenfalls durch Fluor oder Chlor substituiert ist], $C_3$-$C_4$-Alkenyl, $C_1$-$C_2$-Alkoxy-carbonyl-$C_1$-$C_2$-alkyl, Phenyl, Phenylethyl oder Benzyl [welches gegebenenfalls durch Fluor, Chlor, Nitro, Cyano, Methyl, Methoxy, Methoxycarbonyl oder Ethoxycarbonyl substituiert ist] steht,

oder in welcher

(B) M für Wasserstoff oder ein Natrium-, Kalium- oder Calcium-äquivalent steht,

$R^1$ für $C_3$-$C_4$-Alkoxy, $C_1$-$C_3$-Fluoralkoxy, $C_1$-$C_3$-Chloralkoxy, Cyanoethoxy, Methoxyethoxy, $C_1$-$C_2$-Alkoxy-

carbonyl-$C_1$-$C_2$-alkoxy, $C_3$-$C_4$-Alkenoxy, $C_3$-$C_4$-Alkinoxy, Benzyloxy oder Phenoxy, für $C_1$-$C_4$-Alkylamino, Allylamino, Propargylamino, Benzylamino, für Di-($C_1$-$C_2$-alkyl)-amino, für $C_1$-$C_4$-Alkoxyamino, Allyloxyamino, $C_1$-$C_2$-Alkoxy-carbonyl-$C_1$-$C_2$-alkylamino, Phenyl-$C_1$-$C_2$-alkoxyamino, $C_1$-$C_2$-Alkoxy-carbonyl-$C_1$-$C_2$-alkoxyamino, für Dimethylhydrazino oder Phenylhydrazino steht;

in welcher weiter

$R^2$ für Wasserstoff steht und

$R^3$ für den Rest -O-$R^5$ steht, worin

$R^5$ für $C_1$-$C_4$-Alkyl [welches gegebenenfalls durch Fluor oder Chlor substituiert ist], $C_3$-$C_4$-Alkenyl, $C_1$-$C_2$-Alkoxy-carbonyl-$C_1$-$C_2$-alkyl, Phenyl, Phenylethyl oder Benzyl [welches gegebenenfalls durch Fluor, Chlor, Nitro, Cyano, Methyl, Methoxy, Methoxycarbonyl oder Ethoxycarbonyl substiuiert ist7 steht,

sowie - für den Fall, daß M für Wasserstoff steht - die 1:1-Addukte der vorausgehend definierten Verbindungen mit Salzsäure, Schwefelsäure, Trifluoressigsäure, Methansulfonsäure, Benzolsulfonsäure und p-Toluolsulfonsäure.

Verwendet man beispielsweise für die Verfahrensvariante (a) 2-Propoxycarbonyl-benzolsulfonsäurechlorid und N'-(4,6-Dimethyl-pyrimidin-2-yl)-N''-allyloxy-guanidin als Ausgangsstoffe, so kann der Reaktionsablauf durch folgendes Formelschema skizziert werden:

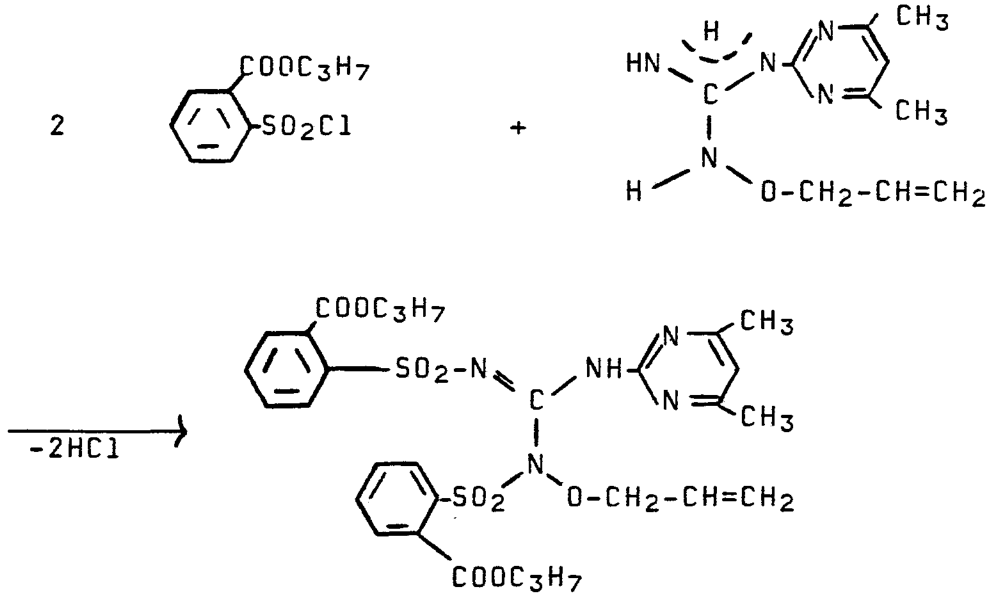


Verwendet man beispielsweise für die Verfahrensvariante(b) N'-(4,6-Dimethyl-pyrimidin-2-yl)-N''-methoxy-N'', N'''-bis-(2-butoxycarbonyl-benzolsulfonyl)-guanidin und Methoxyamin als Ausgangsstoffe, so kann der Reaktionsablauf durch folgendes Formelschema skizziert werden:

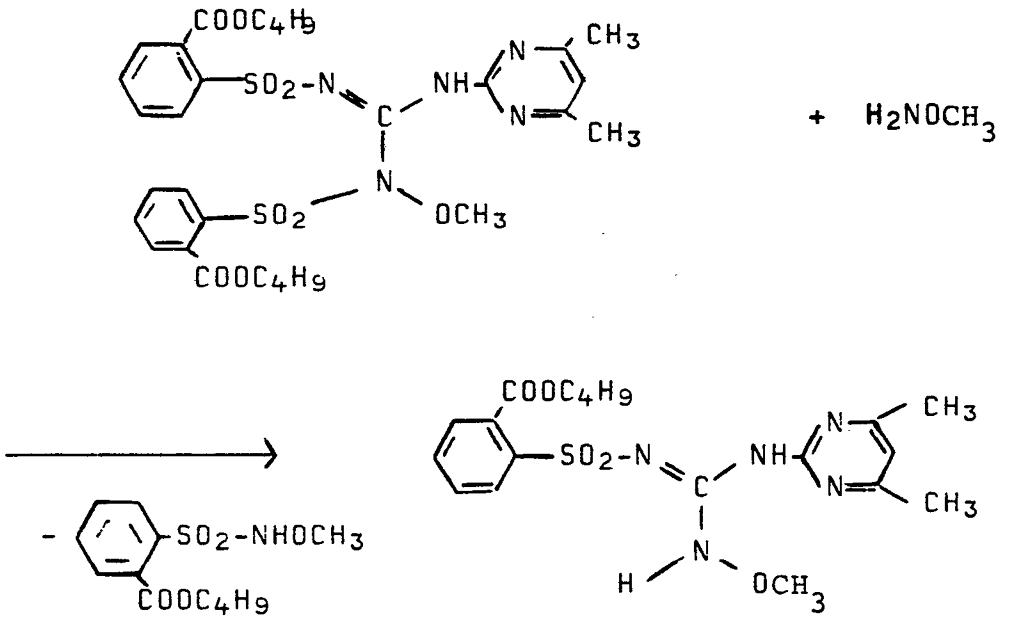

Verwendet man beispielsweise für die Verfahrenvariante (c) das nachstehend angegebene cyclische Diamid und N,N-Dimethylhydrazin als Ausgangsstoffe, so kann der Reaktionsablauf durch folgendes Formelschema skizziert werden:

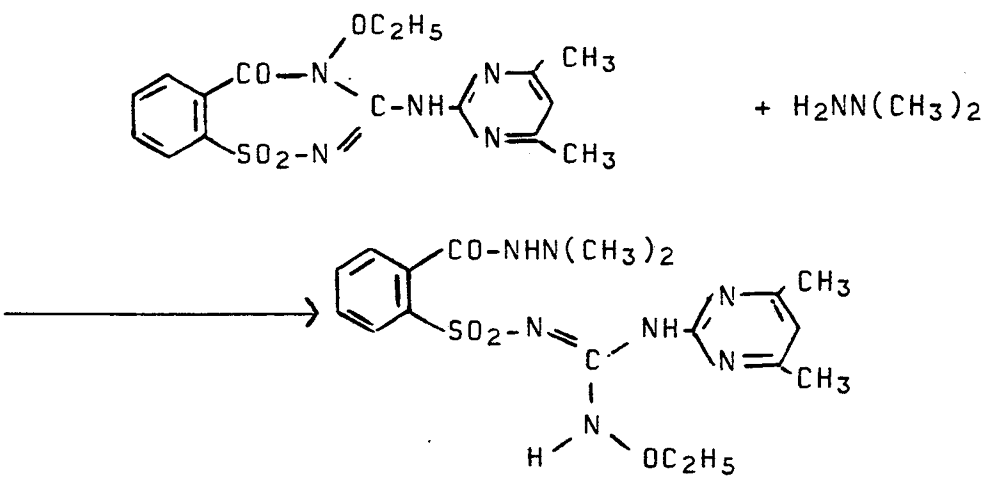

Verwendet man beispielsweise für die Verfahrensvariante (d) N'-(4,6-Dimethyl-pyrimidin-2-yl)-N''-phenoxy-N'''-(2-dimethylaminocarbonyl-benzolsulfonyl)-guanidin und Kaliumethanolat als Ausgangsstoffe, so kann der Reaktionsablauf durch das folgende Formelschema skizziert werden:

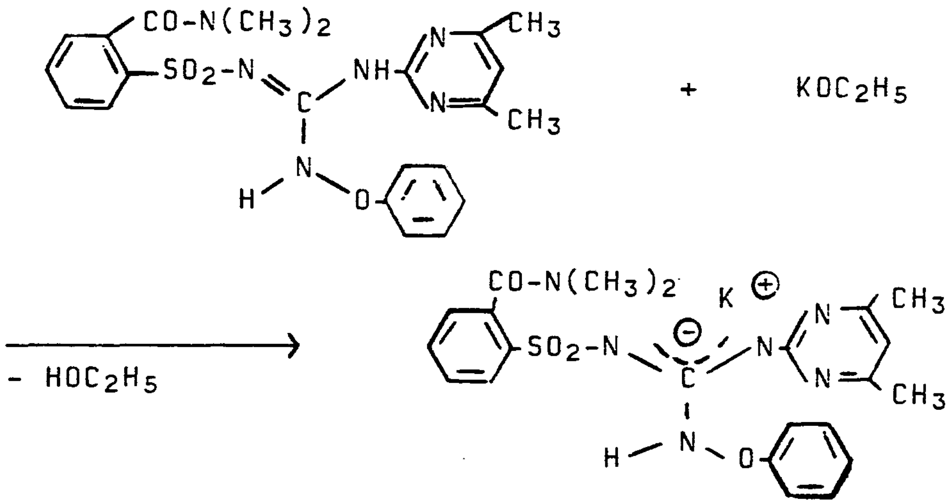


Verwendet man beispielsweise für die Verfahrensvariante (e) N'-(4,6-Dimethyl-pyrimidin-2-yl)-N''-benzyloxy-N'', N'''-bis-(2-ethoxycarbonyl-benzolsulfonyl)-guanidin und Salzsäure als Ausgangsstoffe, so kann der Reaktionsablauf durch folgendes Formelschema skizziert werden:

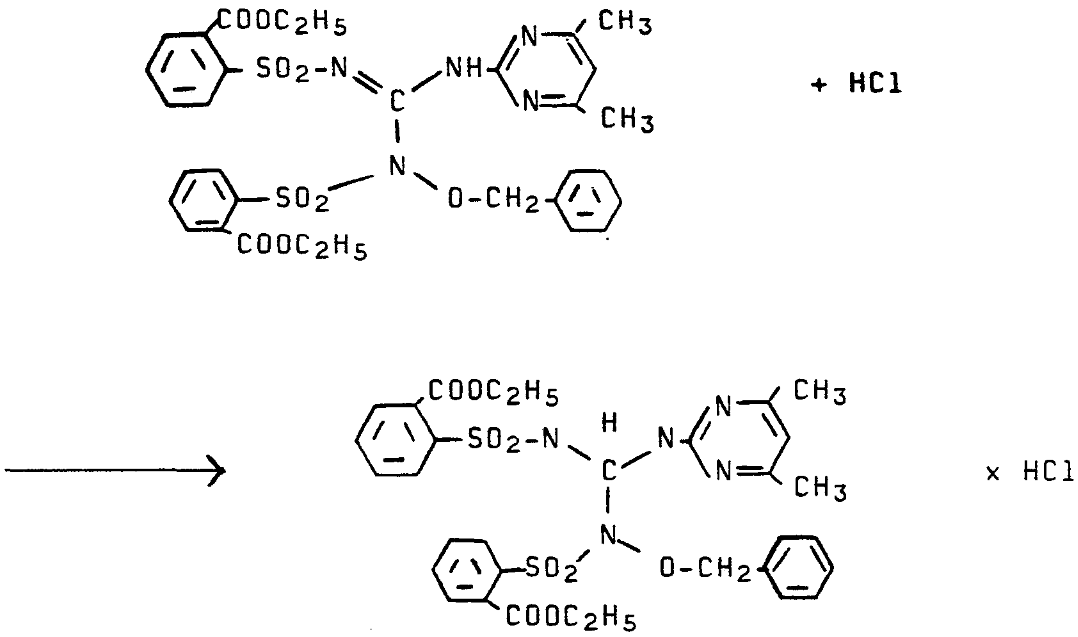


Die als Ausgangsstoffe für die Verfahrensvariante (a) zu verwendenden Guanidin-Derivate sind durch die Formel (II) allgemein definiert. In Formel (II) hat $R^3$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Ausgangsstoffe der Formel (II) seien beispielweise genannt:

N'-(4,6-Dimethyl-pyrimidin-2-yl)-N''-methoxy-guanidin, -N''-ethoxy-guanidin, -N''-propoxy-guanidin,

-N''-isopropoxy-guanidin, -N''-butoxy-guanidin, -N''-isobutoxy-guanidin, -N''-sec.-butoxy-guanidin, -N''-pentoxy-guanidin, -N''-isopentoxy-guanidin, -N''-sec.-pentoxy-guanidin, -N''-hexyloxy-guanidin, -N''-isohexyloxy-guanidin, -N''-heptyloxy-guanidin, -N''-isoheptyloxy-guanidin, -N''-octyloxy-guanidin, -N''-isooctyloxy-guanidin, -N''-allyloxy-guanidin, -N''-crotyloxy-guanidin, -N''-(2-chlor-ethoxy)-guanidin, -N''-(2-fluor-ethoxy)-guanidin, -N''-(2-chlor-propoxy)-guanidin, -N''-(3-chlor-propoxy)-guanidin, -N''-(4-chlor-butoxy)-guanidin, -N''-methoxycarbonylmethyloxy-guanidin, -N''-ethoxycarbonylmethoxy-guanidin, -N''-(1-methoxycarbonyl-ethoxy)-guanidin, -N''-(1-ethoxy-carbonyl-ethoxy)-guanidin, -N''-aminocarbonylmethoxy-guanidin, -N''-(2-phenylethoxy)-guanidin, -N"-phenoxy-guanidin, -N''-(4-methyl-benzyloxy)-guanidin, -N''-(4-fluor-benzyloxy)-guanidin, -N''-(4-chlor-benzyloxy)-guanidin, -N''-(4-nitro-benzyloxy)-guanidin, -N''-(2,6-dichlor-benzyloxy)-guanidin, -N''(4-ethoxy-carbonyl-benzyloxy)-guanidin und -N''-(4-methoxycarbonyl-benzyloxy)-guanidin.

Die Ausgangsstoffe der Formel (II) sind zum Teil bekannt (vergl. J. Chem. Soc. 1962, 3915); zum Teil sind sie Gegenstand einer nicht zum vorveröffentlichten Stand der Technik gehörenden Patentanmeldung der Anmelderin (vergl. DE-OS 3 334 455).

Man erhält die Verbindungen der Formel (II), wenn man 2-Cyanamino-4,6-dimethyl-pyrimidin der Formel (VII)

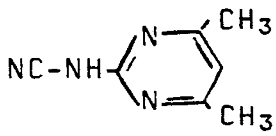

(VII)

mit Aminoverbindungen der Formel (VIII)

$$H_2N-R^3 \qquad (VIII)$$

in welcher

$R^3$ die oben angegebene Bedeutung hat,

bzw. mit deren Hydrochloriden,

gegebenenfalls in Gegenwart von Verdünnungsmitteln, wie z.B. Ethanol, Isopropanol oder Butanol, bei Temperaturen zwischen 20 und 150°C, vorzugsweise zwischen 50 und 120°C, umsetzt und gegebenenfalls die Umsetzungsprodukte mit Säureakzeptoren, wie z.B. Ammoniak, Natronlauge oder Soda, behandelt.

2-Cyanamino-4,6-dimethyl-pyrimidin der Formel (VII) ist bereits bekannt (vergl. J. Chem. Soc. 1953, 1725).

Die Aminoverbindungen der Formel (VIII) sind ebenfalls bereits bekannt und können nach an sich bekannten Verfahren hergestellt werden (vergl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Die weiter als Ausgangsstoffe für die Verfahrensvariante (a) zu verwendenden Arensulfonsäurechloride sind durch die Formel (III) allgemein definiert. In Formel (III) steht vorzugsweise

$R^1$ für $C_3$-$C_6$-Alkoxy, $C_1$-$C_4$-Fluoralkoxy, $C_1$-$C_4$-Chloralkoxy, Cyano-$C_1$-$C_4$-alkoxy, $C_1$-$C_2$-Alkoxy-$C_1$-$C_2$-alkoxy, $C_1$-$C_2$-Alkoxy-carbonyl-$C_1$-$C_2$-alkoxy, $C_3$-$C_5$-Alkenoxy, $C_3$-$C_5$-Alkinoxy, Benzyloxy oder Phenoxy [welches gegebenenfalls durch Fluor, Chlor, Brom, Cyano, Nitro, Methyl, Trifluormethyl, Methoxy, Trifluormethoxy oder Methoxycarbonyl substituiert ist].

In Formel (III) steht insbesondere

$R^1$ für $C_3$-$C_4$-Alkoxy, $C_1$-$C_3$-Fluoralkoxy, $C_1$-$C_3$-Chloralkoxy, Cyanoethoxy,Methoxyethoxy, $C_1$-$C_2$-Alkoxy-carbonyl-$C_1$-$C_2$-alkoxy, $C_3$-$C_4$-Alkenoxy, $C_3$-$C_4$-Alkinoxy, Benzyloxy oder Phenoxy.

Als Ausgangsstoffe der Formel (III) seien beispielsweise genannt:
2-Ethoxycarbonyl-, 2-Propoxycarbonyl-, 2-Isopropoxycarbonyl-, 2-Butoxycarbonyl-, 2-Isobutoxycarbonyl-, 2-sec.-Butoxycarbonyl-, 2-(2-Fluorethoxy)-carbonyl-, 2-(2-Chlorethoxy)-carbonyl-, 2-(2-Cyanoethoxy)-carbonyl-, 2-(2-Methoxy-ethoxy)-carbonyl-, 2-Methoxycarbonylmethoxycarbonyl-, 2-Ethoxycarbonylmethoxycarbonyl-, 2-Allyloxycarbonyl-, 2-Crotyloxycarbonyl-, 2-Propargyloxycarbonyl-, 2-Benzyloxycarbonyl- und 2-Phenoxycarbonyl-benzolsulfonsäurechlorid.

Die Arensulfonsäurechloride der Formel (III) sind bekannt (vgl. Chem Ber. 90 (1957), 841-852; J. Org. Chem. 27 (1962), 1703-1709) und /oder können nach an sich bekannten Verfahren hergestellt werden.

Man erhält die Verbindungen der Formel (III), wenn man Bis-(2-carboxy-phenyl)-disulfid der Formel (IX)

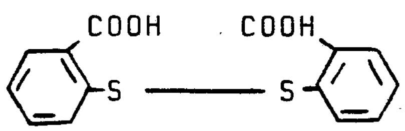

(IX)

mit Thionylchlorid, gegebenenfalls in Gegenwart eines Katalysators, wie z.B. Dimethylformamid, und gegebenenfalls in Gegenwart eines Verdünnungsmittels, wie z.B. Chloroform, bei Temperaturen zwischen 20 und 80°C umsetzt, nach dem Ende der Umsetzung, welches am Aufhören der Gasentwicklung zu erkennen ist, flüchtige Komponenten abdestilliert, den Rückstand, welcher im wesentlichen aus Bis-(2-chlorcarbonyl-phenyl)-disulfid der Formel(X)

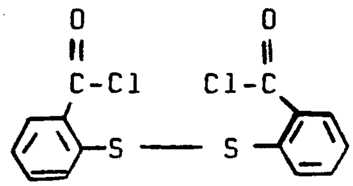

(X)

besteht, mit Verbindungen der Formel (VIa)

$H-R^1$ (VIa)

in welcher

$R^1$ die oben angegebene Bedeutung hat,

gegebenenfalls in Gegenwart eines Säurebindemittels, wie z.B. Triethylamin, und gegebenenfalls in Gegenwart eines Verdünnungsmittels, wie z.B. Toluol oder Methylenchlorid, bei Temperaturen zwischen 0 und 100°C umsetzt, die hierbei gebildeten substituierten Bis-(2-carbonyl-phenyl)-disulfide der Formel (XI)

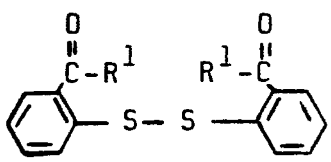

(XI)

in welcher

$R^1$ die oben angegebenen Bedeutung hat,

gegebenenfalls nach Einengen durch Absaugen isoliert, diese Produkte der Formel (XI) in Wasser oder wässriger Salzsäure, oder in einem Gemisch aus Wasser und einem organischen Lösungsmittel, wie z.B. Essigsäure oder Methylenchlorid, dispergiert und bei Temperaturen zwischen 0 und 30°C, gegebenenfalls in Gegenwart eines Katalysators, wie z.B. Tetrabutylammoniumbromid, Chlor bis zur Sättigung einleitet.

Die Aufarbeitung kann nach üblichen Methoden erfolgen, beispielsweise indem man die organische Phase, gegebenenfalls nach Verdünnen mit einem mit Wasser praktisch nicht mischbaren organischen Lösungsmittel, wie z.B. Methylenchlorid, abtrennt, mit Wasser wäscht, trocknet, filtriert, einengt und gegebenenfalls das Produkt durch Destillation unter vermindertem Druck reinigt.

Die als Ausgangsstoffe für die Verfahrensvariante (b) zu verwendenden substituierten Carbonylphenylsulfonylguanidine sind durch die Formel (ID) allgemein definiert. In Formel (ID) haben $R^1$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (ID) seien genannnt:

N'-(4,6-Dimethyl-pyrimidin-2-yl)-N''-methoxy-N'', N'''-bis-(2-ethoxycarbonyl-benzolsulfonyl)-guanidin,-N'', N'''-

bis-(2-propoxycarbonyl-benzolsulfonyl)-guanidin,-N'',N'''-bis-(2-isopropoxycarbonyl-benzolsulfonyl)-guanidin und --N'',N'''-bis-(2-butoxycarbonyl-benzolsulfonyl)-guanidin.

Die Verbindungen der Formel (ID) können nach dem oben unter (a) beschriebenen Verfahren hergestellt werden.

Die bei der Verfahrensvariante (b) weiter als Ausgangsstoffe zu verwendenden Aminoverbindungen sind durch die Formel (IV) allgemein definiert. In Formel (IV) haben $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (IV) seien beispielsweise genannt:

N,N-Dimethylhydrazin, Isopropylhydrazin, Phenylhydrazin, O-Methyl-, O-Ethyl-,

O-Propyl-, O-Isopropyl-, O-Butyl-, O-Isobutyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl-, O-Crotyl-, O-(2-Chlor-ethyl)-, O-(2-Fluorethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4-Fluor-benzyl)-,

O-(4-Chlor-benzyl)-, O-(4-Nitro-benzyl)-, O-(2,6-Dichlor-benzyl)-, O-(4-Methoxycarbonyl-benzyl)- und O-(4-Ethoxy-carbonyl-benzyl)-hydroxylamin.

Aminoverbindungen der Formel (IV) sind bekannt und können nach an sich bekannten Verfahren hergestellt werden(vgl.Chem. Pharm. Bull. 15 (1967), 345; Bull. Soc. Chim. France 1958, 664; Synthesis 1976, 682; J.Chem. Soc.1930, 228 und Helv. Chim. Acta 45 (1962), 1387).

Die als Ausgangsstoffe für die Verfahrensvariante (c) zu verwendenden Verbindungen sind durch die Formel (V) allgemein definiert. In Formel (V) hat $R^3$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Beispiele seien Verbindungen der Formel (V) genannt, in welcher $R^3$ für Methoxy, Ethoxy, Propoxy, Isopropoxy, Butoxy, Isobutoxy, sec.-Butoxy, Benzyloxy, Allyloxy, 3-Chlorpropoxy, Methoxycarbonylmethoxy, 2-Phenylethoxy, 4-Methyl-benzyloxy, 4-Fluorbenzyloxy, 4-Chlor-benzyloxy, 4-Nitro-benzyloxy oder 4-Methoxycarbonyl-benzyloxy steht.

Die Verbindungen der Formel (V) sind noch nicht in der Literatur beschrieben. Man erhält die neuen Verbindungen der Formel (V), wenn man Guanidin-Derivate der Formel (II)

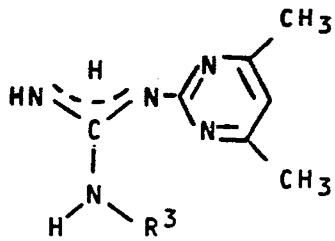

(II)

in welcher

$R^3$ die oben angegebene Bedeutung hat,

mit 2-Chlorsulfonyl-benzoylchlorid der Formel (XII)

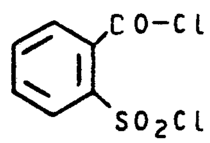

(XII)

gegebenenfalls in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid oder Chloroform, bei Temperaturen zwischen -20°C und +30°C umsetzt.

Die Herstellung der Zwischenprodukte der Formel (II) wurde bereits weiter oben im Zusammenhang mit dem unter (a) angegebenen Verfahren beschrieben.

2-Chlorsulfonyl-benzoylchlorid der Formel (XII) ist bekannt (vgl. DE-OS 2 036 171)

Die bei Verfahren (c) weiter als Ausgangsstoffe zu verwendenden Aminoverbindungen sind durch die Formel (VI) allgemein definiert. In Formel (VI) haben M und $R^1$ vor-

zugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (VI) seien genannt:
Propanol, Isopropanol, Butanol, Isobutanol, sec.-Butanol, 2-Fluor-ethanol, 2-Chlor-ethanol, 3-Chlor-propanol, 2-Cyano-ethanol, 2-Methoxy-ethanol, Glycolsäuremethylester, Allylalkohol, Crotylalkohol, Propargylalkohol, Benzylalkohol, Phenol, Methylamin, Ethylamin, Propylamin, Isopropylamin, Butylamin, Isobutylamin, Allylamin, Propargylamin, Benzylamin, Dimethylamin, Diethylamin, O-Methyl-, O-Ethyl-, O-Propyl-, O-Isopropyl-, O-Butyl-, O-Isobutyl-, O-sec.-Butyl-, O-Allyl-, O-Methoxycarbonylmethyl-, O-Ethoxycarbonylmethyl-, O-(1-Methoxycarbonylethyl)-, O-(1-Ethoxycarbonyl-ethyl)-, O-Phenyl-, O-Benzyl-, O-(2-Phenyl-ethyl)- und O-(4-Methyl-benzyl)-hydroxylamin sowie N,N- Dimethylhydrazin, Isopropylhydrazin, Phenylhydrazin und N,O-Dimethylhydroxylamin.

Die Verbindungen der Formel (VI) sind bekannt und können nach an sich bekannten Verfahren hergestellt werden (vgl. Chem. Pharm. Bull. 15 (1967), 345).

Die bei der Verfahrensvariante (d) als Ausgangsstoffe zu verwendenden substituierten Carbonylphenylsulfonyl-guanidine sind durch die Formel (I) - mit der Maßgabe, daß M für Wasserstoff steht - allgemein definiert. In Formel (I) - soweit sie die als Ausgangsstoffe für Verfahren (d) zu verwendenden Verbindungen betrifft - steht M für Wasserstoff und die Reste $R^1$, $R^2$ und $R^3$ haben vor-

zugsweise bzw. insbesondere die gleichen Bedeutungen, wie sie oben im Rahmen der Substituentendefinition für Formel (I) vorzugsweise bzw. als insbesondere bevorzugt angegeben sind.

Die als Ausgangsstoffe für Verfahren (d) zu verwendenden Verbindungen der Formel (I) können nach den oben unter (a), (b) und (c) beschriebenen Verfahren hergestellt werden.

Als Beispiele für die bei Verfahren (d) zu verwendenden Metallhydroxide, -hydride, -alkanolate bzw. metallorganischen Verbindungen seien genannt: Lithium-, Natrium-, Kalium-, Magnesium- und Calcium-hydroxid, Lithium-, Natrium- und Calcium-hydrid, Natrium-methanolat und -ethanolat, Kalium-methanolat, -ethanolat und Kalium-tert.-butanolat sowie Butyllithium und Isopropylmagnesiumchlorid.

Die bei der Verfahrensvariante (e) als Ausgangsstoffe zu verwendenden Carbonylphenylsulfonyl-guanidine sind durch die Formel (I) - mit der Maßgabe, daß M für Wasserstoff steht - allgemein definiert. In Formel (I) - soweit sie die als Ausgangsstoffe für Verfahren (e) zu verwendenden Verbindungen betrifft - steht M für Wasserstoff und die Reste $R^1$, $R^2$ und $R^3$ haben vorzugsweise bzw. insbesondere die gleichen Bedeutungen, wie sie oben im Rahmen der Substituentendefinition der Formel (I) vorzugsweise bzw. als insbesondere bevorzugt angegeben sind.

Die als Ausgangsstoffe für Verfahren (e) zu verwendenden Verbindungen der Formel (I) können nach den oben unter (a), (b) und (c) beschriebenen Verfahren hergestellt werden.

Bei Verfahren (e) werden starke Säuren als Ausgangsstoffe eingesetzt. Vorzugsweise sind dies Halogenwasserstoffsäuren, wie Hydrogenchlorid, Hydrogenbromid, oder Hydrogeniodid, weiter Schwefelsäure oder gegebenenfalls durch Fluor oder Chlor substituierte Alkansulfonsäuren mit bis zu 4 Kohlenstoffatomen, wie z.B. Methansulfonsäure, Ethansulfonsäure, Chlormethansulfonsäure, 2-Chlorethansulfonsäure und Trifluormethansulfonsäure, Trifluoressigsäure, ferner Benzolsulfonsäure, p-Toluolsulfonsäure, Naphthalin-1,4-, -1,5-, -1,6-, -2,6- und -2,7-disulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls substituierte Kohlenwasserstoffe, wie z.B. Methylenchlorid, Chloroform, 1,2-Dichlorethan, Toluol, Xylol und Chlorbenzol, Nitrile, wie z.B- Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (a) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80 und +100°C, vorzugsweise zwischen -30 und +50°C. Das erfindungsgemäße Verfahren (a) wird im allgemeinen bei Normaldruck durchgeführt.

Zur Durchführung von Verfahren (a) setzt man je Mol Guanidin-Zwischenprodukt der Formel (II) im allgemeinen zwischen 2 und 5 Mol, vorzugsweise zwischen 2,1 und 3 Mol Arensulfonsäurechlorid der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden: Gegebenenfalls nach Abdestillieren flüchtiger Komponenten wird mit Wasser und einem mit Wasser nicht mischbaren Lösungsmittel, wie z.B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z.B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen Lösungsmittel und gegenenenfalls zusätzlich auch Wasser in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester, Nitrile, wie z.B. Acetonitril oder Propionitril, sowie Dimethylformamid und Wasser.

Als Säureakzeptoren können bei Verfahren (b) Säurebindemittel eingesetzt werden, welche in ihren nucleophilen Eigenschaften nicht nennenswert mit den Aminoverbindungen der Formel (IV) konkurrieren.
Als solche seien Alkalimetall- und Erdalkalimetallcarbonate, wie z.B. Kaliumcarbonat und Calciumcarbonat, tertiäre Amine, wie z.B. Triethylamin, N,N-Dimethylanilin und N,N-Dimethylbenzylamin, sowie Stickstoffheterocyclen, wie z.B. Pyridin, Diazabicyclooctan (DABCO) und Diazabicycloundecen (DBU) genannt.

Die Reaktionstemperatur kann bei Verfahren (b) innerhalb eines größeren Bereiches variiert werden. Im allgemeinen arbeitet man zwischen 0°C und 150°C, vorzugsweise zwischen 10°C und 100°C. Verfahren (b) wird im allgemeinen bei Normaldruck durchgeführt.

Zur Durchführung des erfindungsgemäßen Verfahrens (b) setzt man je Mol Guanidin-Derivat der Formel (ID) im allgemeinen zwischen 1 und 10 Mol, vorzugsweise zwischen 2 und 5 Mol Aminoverbindung der Formel (IV) oder deren Hydrochlorid ein.
Im allgemeinen wird das Guanidin-Derivat der Formel (ID) mit dem Verdünnungsmittel bei Raumtemperatur oder unter leichtem Kühlen vorgelegt und die Aminoverbindung der Formel (IV) bzw. das Hydrochlorid hiervon und gegebenenfalls ein geeigneter Säureakzeptor dazugegeben. Das Reaktionsgemisch wird dann im allgemeinen bei Raumtemperatur oder erhöhter Temperatur bis zum Reaktionsende gerührt.

Die Aufarbeitung kann nach üblichen Methoden durchgeführt werden. Soweit die Produkte der Formel (I) aus dem Reaktionsgemisch kristallin anfallen, können sie durch Absaugen isoliert werden. Anderenfalls wird - gegebenenfalls nach Einengen - mit Wasser verdünnt und mit einem mit Wasser praktisch nicht mischbaren Lösungsmittel, wie z.B. Methylenchlorid, extrahiert. Durch Waschen der Extraktionslösung mit Wasser, Trocknen, Filtrieren, Einengen des Filtrates und Umkristallisieren des Rückstandes können die Produkte der Formel (I) in reiner Form erhalten werden.

Das erfindungsgemäße Verfahren (c) zur Herstellung der neuen Verbindungen der Formel (I) wird vorzugsweise unter Verwendung von Verdünnungsmitteln durchgeführt. Als solche kommen praktisch alle inerten organischen Lösungsmittel in Betracht, wie sie oben im Zusammenhang mit der Beschreibung des erfindungsgemäßen Verfahrens (b) genannt wurden.

Die Reaktionstemperatur kann bei Verfahren (c) innerhalb eines größeren Bereiches variiert werden. Im allgemeinen arbeitet man zwischen -20°C und +150°C, vorzugsweise zwischen 0°C und 80 °C. Verfahren (c) wird im allgemeinen bei Normaldruck durchgeführt.

Zur Durchführung von Verfahren (c) setzt man je Mol Verbindung der Formel (V) im allgemeinen zwischen 1 und 3 Mol, vorzugsweise zwischen 1 und 2 Mol Verbindung der Formel (VI) ein.
Im allgemeinen wird die Verbindung der Formel (V) mit dem Verdünnungsmittel vorgelegt und die Verbindung der Formel (VI) - gegebenenfalls im Verdünnungsmittel gelöst - dazugegeben, Das Reaktionsgemisch wird bis zum Reaktionsende gerührt. Die Aufarbeitung kann nach üblichen Methoden durchgeführt werden.

Soweit die Produkte der Formel (I) kristallin anfallen werden sie durch Absaugen isoliert. Andernfalls wird - gegebenenfalls nach Einengen - mit Wasser und einem mit Wasser praktisch nicht mischbaren Lösungsmittel, wie z.B. Methylenchlorid geschüttelt, die organische Phase getrocknet, filtriert und eingeengt. Der Rückstand enthält im wesentlichen die Produkte der Formel (I).

Das erfindungsgemäße Verfahren (d) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z.B. Ethanol, n- und iso-Propanol, Ether, wie z.B. Tetrahydrofuran, Dioxan und 1,2-Dimethoxyethan, Ester, wie z.B. Essigsäureethylester und -methylester sowie Nitrile, wie z.B. Acetonitril.

Die Reaktionstemperatur kann bei Verfahren (d) innerhalb eines größeren Bereiches variiert werden. Im allgemeinen arbeitet man zwischen -20°C und +50°C, vorzugsweise zwischen 0°C und 30°C. Verfahren (d) wird im allgemeinen bei Normaldruck durchgeführt.

Zur Duchführung des erfindungsgemäßen Verfahrens (d) setzt man je Mol Guanidin-Derivat der Formel (I) im allgemeinen zwischen 0,9 und 1,2 Mol, vorzugsweise zwischen 0,95 und 1,1 Mol Metallverbindung ein.

Im allgemeinen werden die Guanidin-Derivate der Formel (I) und das Verdünnungsmittel vorgelegt und - gegebenenfalls unter leichter Außenkühlung - die Metallverbindung - gegebenenfalls im Verdünnungsmittel gelöst - zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (e) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester sowie Ketone, wie Aceton, Methylethylketon und Methylisobutylketon.

Soweit die als Ausgangsstoffe verwendeten Säuren im wässriger Lösung eingesetzt werden, kann vorteilhaft auch Essigsäureanhydrid als Verdünnungsmittel verwendet werden.

Die Reaktionstemperatur kann bei Verfahren (e) innerhalb eines größeren Bereiches variiert werden. Im allgemeinen arbeitet man zwischen -20°C und +50°C, vorzugsweise zwischen 0°C und 30°C. Verfahren (e) wird im allgemeinen bei Normaldruck durchgeführt.

Zur Durchführung des erfindungsgemäßen Verfahrens (e) setzt man je Mol Verbindung der Formel (I) im allgemeinen zwischen 1 und 10 Mol, vorzugsweise 1 und 5 Mol einer starken Säure ein.

Im allgemeinen werden die Verbindungen der Formel (I) und das Verdünnungsmittel vorgelegt und - gegebenenfalls unter leichter Außenkühlung - die starke Säure zudosiert. Das Reaktionsgemisch wird bis zum Reaktionsende gerührt. Die 1:1-Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne

Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-/4-(3,5-Dichlor-pyridin-2-oxy)-phenoxy/-propionsäure-(trimethylsilyl)-methylesters, das R-Enantiomere des 2-/4-(3,5-Dichlorpyridyl-2-oxy)-phenoxy/-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001und 10kg Wirkstoff pro Hektar Bodenfläche, vorzugsweise zwischen 0,05 und 5kg pro ha.
Verbindungen der Formel (I) zeigen auch fungizide Wirkung.

Die Herstellung und die Verwendung der erfindungsgemäßen Wirkstoffe geht aus den nachfolgenden Beispielen hervor.

Herstellungsbeispiele

Beispiel 1

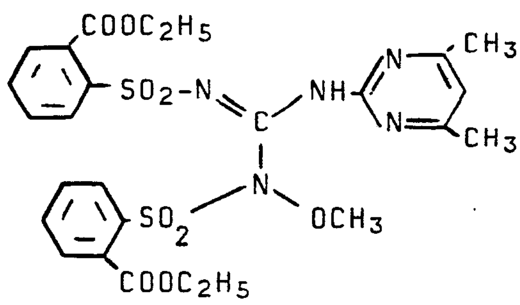

(Verfahren (a))

18,6 g (0,075 Mol) 2-Ethoxycarbonyl-benzolsulfonsäurechlorid werden zu einer auf -10°C gekühlten Mischung aus 4,9g (0,025 Mol) N'-(4,6-Dimethyl-pyrimidin-2-yl)-N''-methoxy-guanidin und 30 ml Pyridin unter Rühren gegeben. Das Reaktionsgemisch wird 3 Tage bei 20°C gerührt und anschließend im Wasserstrahlvakuum eingeengt. Der Rückstand wird in 100 ml Methylenchlorid gelöst, diese Lösung mit Wasser gewaschen, getrocknet, filtriert und eingeengt. Das beim Verreiben des Rückstandes mit Ethanol kristallin angefallene Produkt wird durch Absaugen isoliert.

Man erhält 3,6 g (23% der Theorie) N'-(4,6-Dimethyl-pyrimidin-2-yl)-N''methoxy-N'',N'''-bis-(2-ethoxycarbonyl-benzolsulfonyl)-guanidin vom Schmelzpunkt 106°C.

Beispiel 2

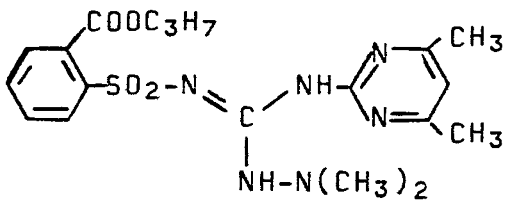

(Verfahren (b))

Eine Mischung aus 18,5 g (0,029 Mol) N'-(4,6-Dimethyl-pyrimidin-2-yl)-N''-methoxy-N'',N'''-bis-(2-propoxycarbonyl-benzolsulfonyl)-guanidin, 4,2 g (0,07 Mol) N,N-Dimethylhydrazin, 40 ml Wasser wird 90 Minuten unter Rückfluß zum Sieden erhitzt und anschließend eingeengt. Der Rückstand wird mit Essigsäureethylester über eine Kieselgelsäule eluiert.

Man erhält 4,8 g (39 % der Theorie) N'-(4,6-Dimethyl-pyrimidin-2-yl)-N''-dimethylamino-N'''-(2-propoxycarbonyl-benzolsulfonyl)-guanidin als gelbes Oel.

Beispiel 3

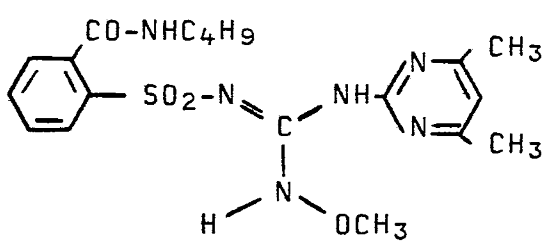

(Verfahren (c))

4,0 g (0,025 Mol) Butylamin werden bei 20-30°C zu einer Mischung aus der Verbindung nachstehender Formel [9,1 g (0,025 Mol)]

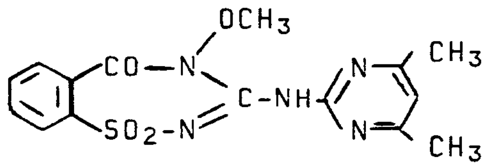

sowie 60ml Diethylether und 5 ml Dioxan unter Rühren gegeben. Das Reaktionsgemisch wird 15 Stunden bei 20-25°C gerührt und das kristallin angefallene Produkt durch Absaugen isoliert. Man erhält 8,1 g (75% der Theorie) N'-(4,6-Dimethyl-pyrimidin-2-yl)-N''-methoxy-N'''-(2-butylaminocarbonyl-benzolsulfonyl)-guanidin vom Schmelzpunkt 169°C.

Beispiel 4

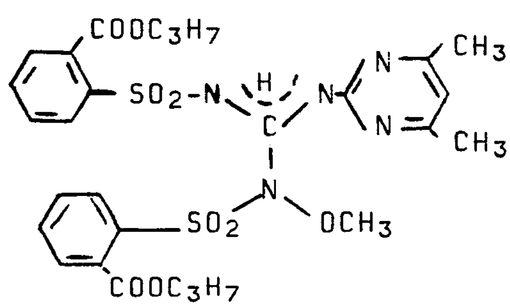

x

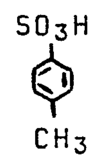

(Verfahren (e))

Eine Mischung aus 4,5 g (0,007 Mol) N'-(4,6-Dimethyl-pyrimidin-2-yl)-N''-methoxy-N''-,N'''-bis-(2-propoxy-carbonylbenzolsulfonyl)-guanidin, 30 ml Aceton und 1,4 g (0,007 Mol) p-Toluolsulfonsäure-hydrat wird 4 Stunden bei 20°C gerührt. Das kristallin angefallene Produkt wird durch Absaugen isoliert.

Man erhält 4,1 g (72% der Theorie) des 1:1-Adduktes aus N'-(4,6-Dimethyl-pyrimidin-2-yl)-N''-methoxy-N'',N'''-bis-(2-propoxycarbonyl-benzolsulfonyl)-guanidin und p-Toluolsulfonsäure vom Schmelzpunkt 172°C.

Nach den in den vorausgehenden Beispielen exemplarisch beschriebenen Verfahren können die in der nachstehenden Tabelle 1 aufgeführten Verbindungen der Formel (I) hergestellt werden:

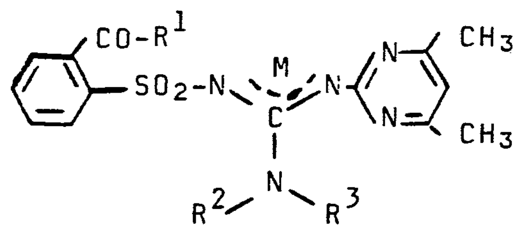

(I)

Tabelle 1

| Beispiel Nr. | M | $R^1$ | $R^2$ | $R^3$ | Schmelz-punkt(°C) |
|---|---|---|---|---|---|
| 5 | H | $-OC_4H_9$-n | 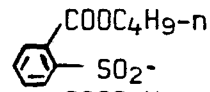 | $-OCH_3$ | 104 |
| 6 | H | $-OC_3H_7$-n | 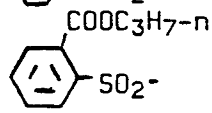 | $-OCH_3$ | 134 |
| 7 | H | $-NHOCH_3$ | H | $-OCH_3$ | 151 |
| 8 | H | $-NHCH_3$ | H | $-OCH_3$ | 177 |
| 9 | H | $-NHN(CH_3)_2$ | H | $-OCH_3$ | 178 |
| 10 | H | $-NHOC_4H_9$-n | H | $-OC_8H_{17}$-n | (amorph) |
| 11 | H | $-OCH(CH_3)_2$ | 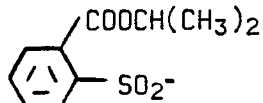 | $-OCH_3$ | 65 |
| 12 | H | $-OC_2H_5$ | 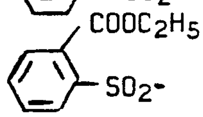 | $-OC_2H_5$ | |
| 13 | H | $-OC_4H_9$-n | 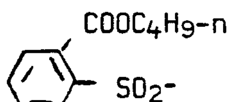 | $-OC_2H_5$ | |
| 14 | H | $-OC_3H_7$-n | 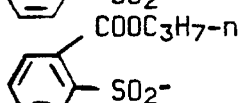 | $-OC_2H_5$ | |
| 15 | H | $-OCH(CH_3)_2$ | 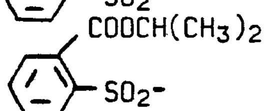 | $-OC_2H_5$ | 96 |
| 16 | H | $-OC_2H_5$ | 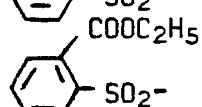 | 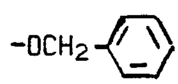 | 135 |
| 17 | H | $-OC_4H_9$-n | 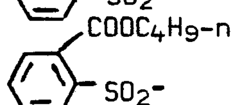 | 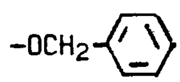 | |
| 18 | H | $-OC_3H_7$-n | 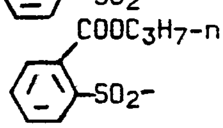 | 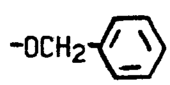 | |

Tabelle 1-Fortsetzung

| Beispiel Nr | M | $R^1$ | $R^2$ | $R^3$ | Schmelzpunkt (°C) |
|---|---|---|---|---|---|
| 19 | H | $-OCH(CH_3)_2$ | 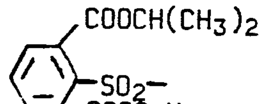 | $-OCH_2-$ 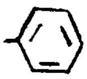 | 120 |
| 20 | H | $-OC_2H_5$ | 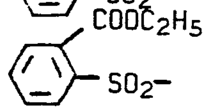 | $-OCH_2-CH=CH_2$ | |
| 21 | H | $-OC_4H_9-n$ | 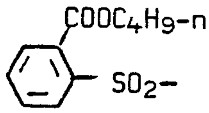 | $-OCH_2-CH=CH_2$ | |
| 22 | H | $-OC_3H_7-n$ | 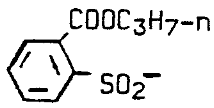 | $-OCH_2-CH=CH_2$ | |
| 23 | H | $-OCH(CH_3)_2$ | 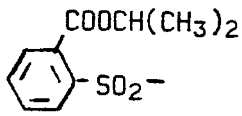 | $-OCH_2-CH=CH_2$ | |
| 24 | H | $-OC_2H_5$ | 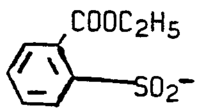 | $-OCH_2COOC_2H_5$ | |
| 25 | H | $-OC_4H_9-n$ | 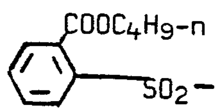 | $-OCH_2COOC_2H_5$ | |
| 26 | H | $-OC_3H_7-n$ | 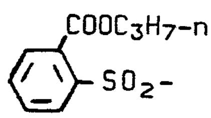 | $-OCH_2COOC_2H_5$ | |
| 27 | H | $-N(CH_3)_2$ | 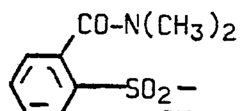 | $-OCH_3$ | 153 |
| 28 | H | 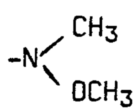 | 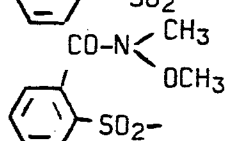 | $-OCH_3$ | |
| 29 | H | 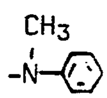 | 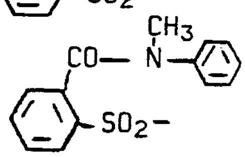 | $-OCH_3$ | |

Tabelle 1 - Fortsetzung

| Beispiel Nr. | M | $R^1$ | $R^2$ | $R^3$ | Schmelzpunkt (°C) |
|---|---|---|---|---|---|
| 30 | H | $-OH$ | H | $-OCH_3$ | |
| 31 | H | $-OCH(CH_3)_2$ | H | $-N(CH_3)_2$ | 142 |
| 32 | H | $-OC_4H_9$-n | H | $-N(CH_3)_2$ | 110 |
| 33 | H | $-NHOC_3H_7$ | H | $-OCH_3$ | 138 |
| 34 | H | $-NHOCH(CH_3)_2$ | H | $-OCH_3$ | 178 |
| 35 | H | $-NHOCH_3$ | H | $-OCH_2-$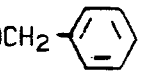 | |
| 36 | H | $-NHOCH_3$ | H | $-OCH(CH_3)_2$ | |
| 37 | H | $-NHOCH_2-$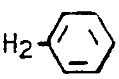 | H | $-OCH_3$ | |
| 38 | H | $-OCH(CH_3)_2$ | H | $-OH$ | |
| 39 | H | $-OC_4H_9$ | H | $-NH-$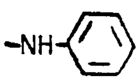 | |
| 40 | H | $-N(CH_3)_2$ | H | $-OC_2H_5$ | 143-148 |
| 41 | H | $-NHOCH_3$ | H | $-OC_4H_9$-n | (amorph) |
| 42 | H | $-NHOC_4H_9$-n | H | $-OCH_2-$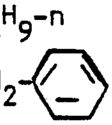 | (amorph) |
| 7a | 1:1-Addukt von Verbindung Nr. 7 mit p-Toluolsulfonsäure | | | | 189 |
| 7b | 1:1-Addukt von Verbindung Nr. 7 mit Schwefelsäure | | | | (amorph) |
| 8a | 1:1-Addukt von Verbindung Nr. 8 mit Schwefelsäure | | | | 160 (Zers.) |

Tabelle 1 (Fortsetzung)

| Beispiel Nr. | M | $R^1$ | $R^2$ | $R^3$ | Schmelzpunkt (°C) |
|---|---|---|---|---|---|
| 43 | H | $-NH_2$ | -H | $-OCH_3$ | 222 |
| 44 | H | $-N(CH_3)_2$ | -H | $-OC_2H_5$ | 143-148 |
| 45 | H | -OH | -H | $-OCH_2-$ 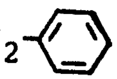 | 85 |
| 46 | H | $-OC_2H_5$ | -H | $-N(CH_3)_2$ | Öl |
| 47 | H | $-OCH_2CH_2Cl$ | 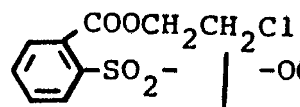 | $-OCH_3$ | 136 |
| 48 | H | $-OCH_2CH_2Cl$ | -H | $-N(CH_3)_2$ | 128 |
| 49 | H | $-OCH_2CH_2CH_3$ | 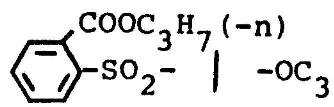 | $-OC_3H_7(-n)$ | 127 |
| 50 | H | $-OCH_2CH_2Cl$ | 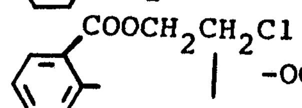 | $-OC_3H_7(-n)$ | 104-106 |
| 51 | H | $-OCH_2CH_2Cl$ | 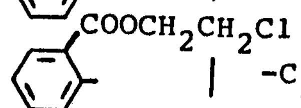 | $-C_2H_5$ | 142 |
| 52 | H | $-OCH_2CH_2Cl$ | 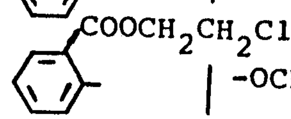 | $-OCH_2CH=CH_2$ | 128 |

Tabelle 1 (Fortsetzung)

| Beispiel Nr. | | Schmelzpunkt (°C) |
|---|---|---|
| 16a | 1:1-Addukt von Verbindung Nr. 16 mit Methansulfonsäure | 96 |
| 16b | 1:1-Addukt von Verbindung Nr. 16 mit Schwefelsäure | 74 |
| 19a | 1:1-Addukt von Verbindung Nr. 19 mit Methansulfonsäure | 107 |

Herstellung von Ausgangsstoffen der Formel (II)

Beispiel (II-1)

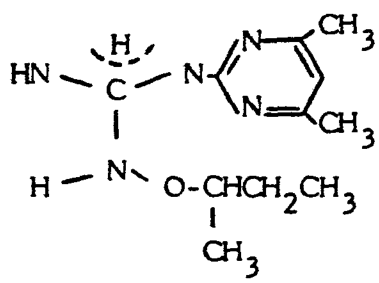

Eine Mischung aus 143 g (0,97 Mol) 2-Cyanamino-4,6-dimethyl-pyrimidin, 94,3 g (1,06 Mol) O-sec-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57% der Theorie) N'-(4,6-Dimethyl-pyrimidin-2-yl)-N"-(1-methyl-propoxy)-guanidin vom Schmelzpunkt 52°C.

Beispiel (II-2)

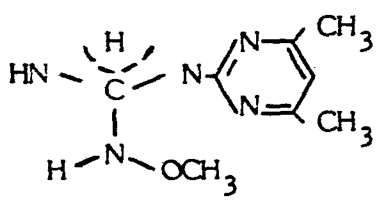

Eine Mischung aus 109 g (0,67 Mol) O-Methylhydroxylamin-Hydrochlorid, 99 g (0,67 Mol) 2-Cyanamino-4,6-dimethyl-pyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55% der Theorie) N'-(4,6-Dimethyl-pyrimidin-2-yl)-N"-methoxy-guanidin vom Schmelzpunkt 134°C bis 136°C.

Analog können die in der nachstehenden Tabelle 2 aufgeführten Verbindungen der Formel (II) hergestellt werden:

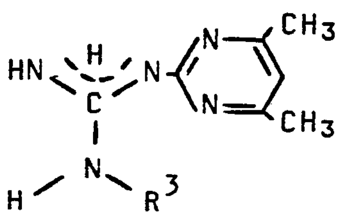

(II)

Tabelle 2:

| Bsp. Nr. | $R^3$ | Schmelzpunkt (°C) |
|---|---|---|
| II-3 | $-OCH_2CH(CH_3)_2$ | 78 |
| II-4 | $-OCH_2CH{=}CH_2$ | 103 |
| II-5 | $-OCH(CH_3)_2$ | 84 |
| II-6 | $-OCH_2CH_2-$ 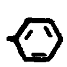 | Öl |

Tabelle 2 - Fortsetzung

| Beispiel Nr. | $R^3$ | Schmelzpunkt(°C) |
|---|---|---|
| II-7 | $-OC_4H_9-n$ | Oel |
| II-8 | $-OC_8H_{17}-n$ | 58 |
| II-9 | $-OCH_2CH_2CH_2Cl$ | 137 |
| II-10 | 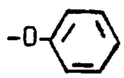 | 192 (Zers.) |
| II-11 | $-OCH_2-COOCH_3$ | 148-149 |
| II-12 | $-O-CH_2-COOC_2H_5$ | 98-99 |
| II-13 | $-O-CH(CH_3)-COOCH_3$ | 147-148 |
| II-14 | 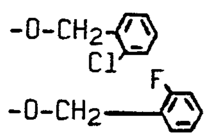 | 103 |
| II-15 | | 114-116 |
| II-16 | 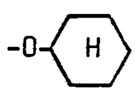 | |
| II-17 | 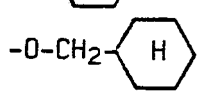 | |
| II-18 | $-O-CH_2CON(CH_3)_2$ | |
| II-19 | 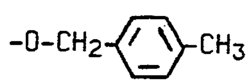 | 86 |
| II-20 | $-O-CH_2-O-CH_3$ | |
| II-21 | $-O-CH_2-S-CH_3$ | |
| II-22 | 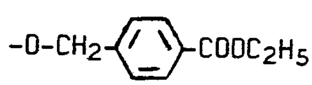 | 138 |

Tabelle 2 - Fortsetzung

| Beispiel Nr. | $R^3$ | Schmelzpunkt(°C) |
|---|---|---|
| II-23 | -O-$CH_2$ 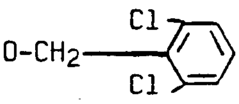 | 145 |
| II-24 | -O-$CH_2$- 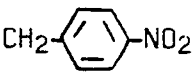 | 172 |
| II-25 | $-OC_2H_5$ | 88 |
| II-26 | -O-$CH_2$- 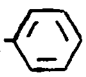 | 102 |
| II-27 | $-O-C_3H_7$(-n) | 54 |
| II-28 | $-O-CH_2-COOC_3H_7$(-n) | 112 |
| II-29 | $-O-CH_2-CF_3$ | |

Herstellung von Ausgangsstoffen der Formel (III)

Beispiel (III-1)

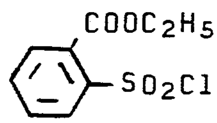

Eine Mischung aus 100 g (0,33 Mol) Bis-(carboxy-phenyl)-disulfid und 100 ml Thionylchlorid wird langsam auf 70°C erhitzt und bei dieser Temperatur gehalten, bis die Gasentwicklung praktisch beendet ist. Anschließend wird eingeengt. Vom hierbei als Rückstand verbliebenen rohen Bis-(2-chlorcarbonyl-phenyl)-disulfid - 101 g (91% der Theorie) - werden 50 g (0,14 Mol) entnommen und mit 100 ml Ethanol 3 Stunden unter Rückfluß zum Sieden erhitzt. Das nach Erkalten kristallin angefallene Bis-(2-ethoxycarbonyl-phenyl)-disulfid - 41 g (89% der Theorie), Schmelzpunkt 103°C - wird durch Absaugen isoliert und in 100 ml Wasser und 100 ml Methylenchlorid aufgenommen. Nach Zugabe von 1 g Tetrabutylammoniumbromid wird bei einer Innentemperatur zwischen 0°C und 10°C Chlor bis zur Sättigung eingeleitet. Dann wird die organische Phase abgetrennt, mit Wasser gewaschen, getrocknet, filtriert und eingeengt.

Man erhält 48 g (84% der Theorie) 2-Ethoxycarbonyl-benzolsulfonsäurechlorid (nach GC 97%ig) als gelbes Oel.

Herstellung von Ausgangsstoffen der Formel (V)

Beispiel (V -1)

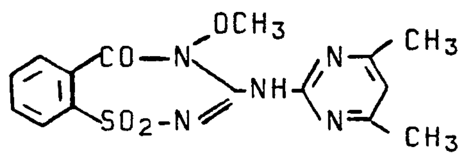

Eine Lösung von 60 g (0,25 Mol) 2-Chlorsulfonylbenzoylchlorid wird unter Rühren bei -10°C bis -5°C zu einer Mischung von 49 g (0,25 Mol) N'-(4,6-Dimethylpyrimidin-2-yl)-N''-methoxyguanidin, 50 g (0,63 Mol) Pyridin und 200 ml Methylenchlorid getropft.
Man rührt dann 3 Stunden bei 20 bis 25°C nach. Nach dem Waschen der Methylenchloridlösung mit verdünnter Salzsäure und Eiswasser, Trocknen, Filtrieren und Einengen erhält man einen Rückstand der durch Verreiben mit Aethanol zur Kristallisation gebracht wird. Das hierbei kristallin angefallene Produkt wird durch Absaugen isoliert.

Man erhält 65 g (72% der Theorie) der Verbindung der oben angegebenen Strukturformel vom Schmelzpunkt 185°C.

Nach dem im vorausgehenden Beispiel exemplarisch beschriebenen Verfahren können die in der nachstehenden Tabelle 3 aufgeführten Verbindungen der Formel (V) hergestellt werden.

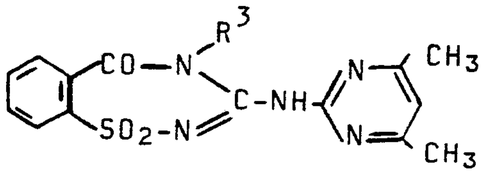

(V)

Tabelle 3

| Beispiel Nr. | $R^3$ | Schmelzpunkt(°C) |
|---|---|---|
| V-2 | $-O-CH_2-$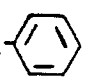 | 162 |
| V-3 | $-O-C_8H_{17}-n$ | 136 |
| V-4 | $-O-C_4H_9-n$ | (amorph) |
| V-5 | $-OC_2H_5$ | 175 |
| V-6 | $-OC_3H_7-n$ | 81 |
| V-7 | $-O-CH(CH_3)_2$ | 155 |
| V-8 | $-OCH_2CH(CH_3)_2$ | |
| V-9 | $-O-CH_2CH_2-$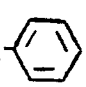 | |
| V-10 | $-O-CH_2-COOC_2H_5$ | |
| V-11 | $-O-CH-CH_2CH_3$<br>\|<br>$CH_3$ | |
| V-12 | $-O-CH_2-CH=CH_2$ | 153-155 |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirstoffe zeigen in diesem Test eine sehr gute herbizide Wirksamkeit. Dies gilt in besonderem Maße z.B. für die Verbindungen gemäß Herstellungsbeispielen 1, 4, 6.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Dies gilt in besonderem Maße z.B. für die Verbindungen gemäß Herstellungsbeispielen 1, 4, 6.

Patentansprüche

1) Substituierte Carbonylphenylsulfonyl-guanidine der allgemeinen Formel (I)

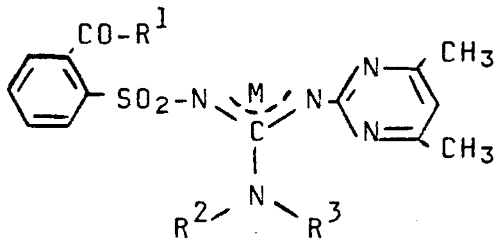

(I)

in welcher

M für Wasserstoff oder ein Metalläquivalent steht,

$R^1$ für Hydroxy, $C_3$-$C_8$-Alkoxy und - für den Fall, daß $R^2$ von Wasserstoff verschieden ist - auch für Ethoxy steht und weiter für $C_3$-$C_6$-Cycloalkoxy, $C_3$-$C_6$-Cycloalkyl-$C_1$-$C_2$-alkoxy, $C_1$-$C_6$-Halogenalkoxy, Cyano-$C_1$-$C_6$-alkoxy, $C_1$-$C_4$-Alkoxy-$C_1$-$C_4$-alkoxy, $C_1$-$C_4$-Alkylthio-$C_1$-$C_4$-alkoxy, $C_1$-$C_4$-Alkylsulfinyl-$C_1$-$C_4$-alkoxy, $C_1$-$C_4$-Alkylsulfonyl-$C_1$-$C_4$-alkoxy, $C_1$-$C_4$-Alkoxy-carbonyl-$C_1$-$C_2$-alkoxy, $C_3$-$C_8$-Alkenoxy, $C_3$-$C_8$-Alkinoxy, Phenyl-$C_1$-$C_3$-alkoxy, Phenoxy [welches gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy oder $C_1$-$C_4$-Alkoxycarbonyl substituiert ist], für $C_1$-$C_8$-Alkylamino welches gegebenenfalls durch Halogen, Hydroxy, Cyano, $C_1$-$C_4$-Alkoxy oder

$C_1$-$C_4$-Alkoxy-carbonyl substituiert ist], $C_3$-$C_6$-Cycloalkylamino, $C_3$-$C_6$-Cyclo alkyl-$C_1$-$C_2$-alkylamino,$C_3$-$C_8$-Alkenylamino, $C_3$-$C_8$-Alkinylamino, Phenyl-$C_1$-$C_3$-alkylamino, für Di-($C_1$-$C_4$-alkyl)-amino [welches gegebenenfalls durch Halogen,Cyano Hydroxy oder $C_1$-$C_4$-Alkoxy substituiert ist], Di-($C_3$-$C_6$-Cycloalkylamino), Di-($C_3$-$C_5$-alkenyl )-amino, Di-($C_3$-$C_5$-alkinyl)-amino, für $C_1$-$C_8$-Alkoxyamino [welches gegebenenfalls durch Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_8$-Alkenyloxyamino ,$C_3$-$C_8$-Alkinyloxyamino, $C_3$-$C_6$-Cycloalkyloxyamino, $C_1$-$C_4$-Alkoxy-carbonyl-$C_1$-$C_2$-alkoxyamino, Phenyl-$C_1$-$C_2$- alkoxyamino [welches gegebenenfalls durch Halogen, Cyano, Nitro, $C_1$-$C_4$-Alkyl, $C_1$-$C_4$-Alkoxy oder $C_1$-$C_4$-Alkoxy-carbonyl substituiert ist], für $C_1$-$C_6$-Alkylhydrazino, Di-($C_1$-$C_4$-alkyl)-hydrazino, $C_3$-$C_6$-Cycloalkylhydrazino, Phenyl-$C_1$-$C_2$-alkylhydrazino, Phenylhydrazino [welches gegebenenfalls durch Halogen, Cyano, Nitro,$C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-Alkoxy-carbonyl substituiertist], für $C_1$-$C_4$-Alkyl-carbonyl-hydrazino, $C_1$-$C_4$-Alkoxy-carbonyl-hydrazino, $C_1$-$C_4$-Alkyl-sulfonylhydrazino, $C_1$-$C_4$-Halogenalkylsulfonylhydrazino oder Phenylsulfonylhydrazino [welches gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy oder $C_1$-$C_4$-Alkoxy-carbonyl substituiert ist] oder für Amino steht;

in welcher weiter

$R^2$ für Wasserstoff, $C_1$-$C_6$-Alkyl [welches gegebenenfalls durch Fluor, Chlor, Brom Cyano, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für den Rest

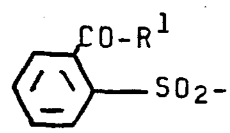

steht, worin

$R^1$ die oben angegebene Bedeutung hat;

in welcher weiter

$R^3$ für Wasserstoff, $C_1$-$C_6$-Alkyl [welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht, oder

$R^2$ und $R^3$ gemeinsam für $C_4$-$C_6$-Alkandiyl stehen, /welches gegebenenfalls durch eine Sauerstoff-Brücke oder durch eine Brücke >N-$R^4$ unterbrochen ist/, wobei

$R^4$ für $C_1$-$C_4$-Alkyl, $C_1$-$C_4$-Alkyl-carbonyl, $C_1$-$C_4$-Alkoxy-carbonyl oder Phenyl steht /welches gegebenenfalls durch Fluor, Chlor, Brom, Cyano, Nitro, $C_1$-$C_4$-Alkyl, $C_1$-$C_2$-Fluoralkyl oder $C_1$-$C_4$-Alkoxy substituiert ist/;

in welcher weiter

$R^3$ für den Rest -O-$R^5$ steht, worin

$R^5$ für $C_1$-$C_8$-Alkyl /welches gegebenenfalls durch Halogen, $C_1$-$C_4$-Alkoxy, $C_1$-$C_4$-Alkylthio, $C_1$-$C_4$-Alkylsulfonyl substituiert ist/, $C_3$-$C_6$-Alkenyl, /welches gegebenenfalls durch Fluor oder Chlor substituiert ist/, $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonylmethyl, $C_1$-$C_4$-Alkylamino-carbonyl-methyl, Di-($C_1$-$C_4$-alkyl)-amino-carbonyl-methyl, Phenyl, Benzyl oder Phenylethyl /welche gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy oder $C_1$-$C_4$-Alkoxycarbonyl substituiert sind / steht;

in welcher weiter

$R^3$ für den Rest $-N\begin{matrix} R^6 \\ R^7 \end{matrix}$ steht, worin

$R^6$ für Wasserstoff oder $C_1$-$C_4$-Alkyl steht und

$R^7$ für $C_1$-$C_4$-Alkyl [welches gegebenenfalls durch Halogen, Cyano, Nitro, $C_1$-$C_4$-Alkoxy oder $C_1$-$C_4$-Alkoxy-carbonyl substituiert ist], $C_3$-$C_6$-Cycloalkyl, Phenyl-$C_1$-$C_2$-alkyl, Phenyl [welches gegebenenfalls durch Halogen,Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-Alkoxy-carbonyl substituiert ist], für $C_1$-$C_4$-Alkyl-carbonyl, $C_1$-$C_4$-Alkoxy-carbonyl, $C_1$-$C_4$-Alkylsulfonyl, $C_1$-$C_4$-Halogenalkylsulfonyl oder Phenylsulfonyl [welches gegebenenfalls durch Halogen, Cyano,Nitro, $C_1$-$C_4$-Alkyl, $C_1$-$C_4$-Halogenalkyl, $C_1$-$C_4$-Alkoxy, $C_1$-$C_2$-Halogenalkoxy oder $C_1$-$C_4$-Alkoxy-carbonyl substituiert ist] steht,

sowie 1:1-Addukte von Verbindungen der Formel (I) mit starken Säuren.

2) Verfahren zur Herstellung von substituierten Carbonylphenylsulfonylguanidinen der allgemeinen Formel (I) gemäß Anspruch 1, dadurch gekennzeichnet, daß man

(a) für den Fall, daß M für Wasserstoff steht und $R^2$ für den Rest 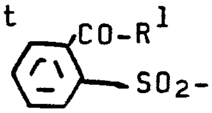 steht,

Guanidin-Derivate der Formel (II)

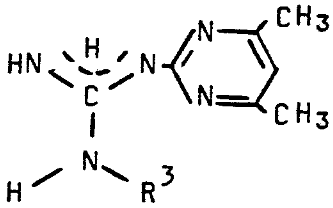 (II)

in welcher

$R^3$ die oben angegebene Bedeutung hat,

mit wenigstens zwei Moläquivalenten von substituierten Arensulfonsäurechloriden der Formel (III)

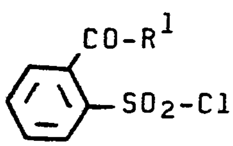 (III)

in welcher

$R^1$ die oben angegebene Bedeutung hat,

in Gegenwart von Säureakzeptoren und gegebenenfalls in Gegenwart von Verdünnungsmitteln umsetzt;

oder daß man

(b) für den Fall, daß M für Wasserstoff steht und $R^2$ für Wasserstoff, $C_1$-$C_6$-Alkyl [welches gegebenenfalls wie oben angegeben substituiert ist], $C_3$-$C_6$-Cycloalkyl, $C_3$-$C_6$-Alkenyl, $C_3$-$C_6$-Alkinyl oder Benzyl [welches gegebenenfalls wie oben angegeben substituiert ist] steht oder zusammen mit $R^3$ die oben angegebene Bedeutung hat,

die nach dem unter (a) angegebenen Verfahren erhältlichen substituierten Carbonylphenylsulfonylguanidine der Formel (ID)

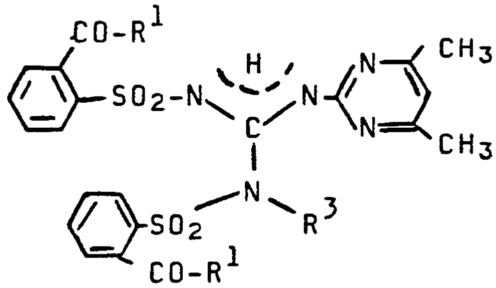

(ID)

in welcher

$R^1$ und $R^3$ die oben angegebenen Bedeutungen haben,

mit Aminoverbindungen der Formel (IV)

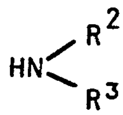 (IV)

in welcher

$R^2$ die vorausgehend unter (b) angegebenen Bedeutung hat und

$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder daß man

(c) für den Fall, daß M und $R^2$ für Wasserstoff stehen, Verbindungen der Formel (V)

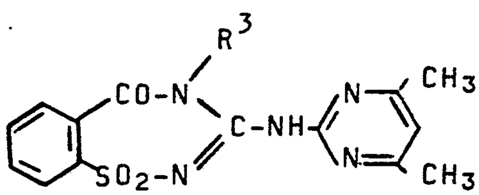 (V)

in welcher

$R^3$ die oben angegebene Bedeutung hat

mit Verbindungen der Formel (VI)

$$M-R^1 \qquad (VI)$$

in welcher

M und $R^1$ die oben angegebenen Bedeutungen haben

gegebenenfalls in Gegenwart von Verdünnungsmitteln umsetzt;

oder daß man

(d) für den Fall, daß M für ein Metalläquivalent steht, die nach den oben unter (a), (b) und (c) angegebenen Verfahren erhältlichen Verbindungen der Formel (I) in welcher

M für Wasserstoff steht und

$R^1$, $R^2$ und $R^3$ die oben angegebenen Bedeutungen haben,

mit Metallhydroxiden, -hydriden oder -alkolaten oder mit metallorganischen Verbindungen gegebenenfalls in Gegenwart von Verdünnungsmitteln umsetzt;

oder daß man

(e) für den Fall, daß 1:1-Addukte von Verbindungen der Formel (I) mit starken Säuren herzustellen sind,

Verbindungen der Formel (I) in welcher

M für Wasserstoff steht und

$R^1$, $R^2$ und $R^3$ die oben angegebenen Bedeutungen haben,

mit starken Säuren gegebenenfalls in Gegenwart von Verdünnungsmitteln umsetzt.

3) Herbizide Mittel, gekennzeichnet durch einen Gehalt an mindestens einem substituierten Carbonylphenylsulfonylguanidin der allgemeinen Formel (I) gemäß Anspruch 1.

4) Verwendung von substituierten Carbonylphenylsulfonylguanidinen der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man substituierte Carbonylphenylsulfonylguanidine der allgemeinen Formel (I) gemäß Anspruch 1 mit Streckmitteln und/oder oberflächenaktiven Mitteln vermischt.

6) Verbindungen der allgemeinen Formel (V)

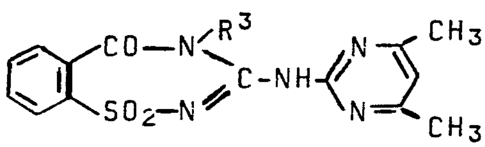

(V)

in welcher

$R^3$ für Wasserstoff, Hydroxy, $C_1$-$C_4$-Alkyl [welches gegegebenenfalls durch Fluor, Chlor, Brom, Cyano, Carboxy, $C_1$-$C_3$-Alkoxy-carbonyl, Hydroxy oder $C_1$-$C_2$-Alkoxy substituiert ist], $C_3$-$C_6$-Cycloalkyl, $C_3$-$C_5$-Alkenyl, $C_3$-$C_5$-Alkinyl, Phenylethyl, Benzyl [welches gegebenenfalls durch Fluor, Chlor, Nitro, Cyano, Methyl, Methoxy oder Methoxycarbonyl substituiert ist] oder Phenyl [ welches gegebenenfalls durch Fluor, Chlor, Brom, Hydroxy, Cyano, Nitro, Amino, $C_1$-$C_3$-Alkyl, Trifluormethyl, $C_1$-$C_3$-Alkoxy, Trifluormethoxy, $C_1$-$C_3$-Alkylthio, Trifluormethylthio, Aminosulfonyl oder $C_1$-$C_2$-Alkoxy-carbonyl substituiert ist] steht, oder

in welcher weiter

$R^3$ für den Rest $-O-R^5$ steht, worin

$R^5$ für $C_1$-$C_8$-Alkyl [welches gegebenenfalls durch Fluor oder Chlor substituiert ist], $C_3$-$C_6$-Alkenyl, $C_1$-$C_3$-Alkoxy-carbonyl-$C_1$-$C_2$-alkyl, Phenyl, Phenylethyl oder Benzyl [welches gegebenenfalls

durch Fluor, Chlor, Nitro, Cyano, $C_1$-$C_2$-Alkyl, $C_1$-$C_2$-Alkoxy oder $C_1$-$C_2$-Alkoxy-carbonyl substituiert ist] steht;

in welcher weiter

$R^3$ für den Rest 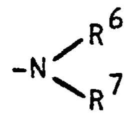 steht, worin

$R^6$ für Wasserstoff oder Methyl steht und

$R^7$ für $C_1$-$C_2$-Alkyl [welches gegebenenfalls durch Fluor, Chlor, Cyano, $C_1$-$C_2$-Alkoxy oder $C_1$-$C_2$-Alkoxy-carbonyl substituiert ist], $C_3$-$C_6$-Cycloalkyl, Phenylethyl, Benzyl oder Phenyl [welches gegebenenfalls durch Fluor, Chlor, Brom, Nitro, Cyano, $C_1$-$C_4$-Alkyl, $C_1$-$C_4$-Alkoxy oder $C_1$-$C_2$-Alkoxy-carbonyl substituliert ist] steht.

7) Verfahren zur Herstellung von Verbindungen der allgemeinen Formel (V) gemäß Anspruch 6, dadurch gekennzeichnet, daß man Guanidin-Derivate der allgemeinen Formel (II)

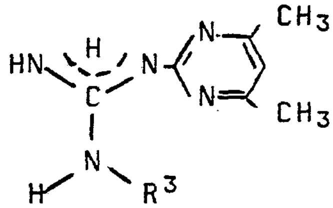

(II)

in welcher

$R^3$ die oben angegebene Bedeutung hat,

mit 2-Chlorsulfonyl-benzylchlorid der Formel (XII)

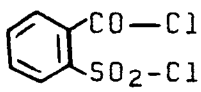

(XII)

gegebenenfalls in Gegenwart von Säureakzeptoren und gegebenenfalls in Gegenwart von Verdünnungsmitteln umsetzt.

Europäisches Patentamt

**0 173 320**

# EUROPÄISCHER RECHERCHENBERICHT

EP 85110832.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | EP - A1 - 0 117 014 (E.I. DU PONT DE NEMOURS)<br>* Zusammenfassung; Formel II; Tabellen 23, 25 *<br>-- | 1,3-5 | C 07 D 239/42<br>C 07 D 417/12<br>A 01 N 47/44 |
| D,P | DE - A1 - 3 334 455 (BAYER AG) | | |
| Y | * Patentansprüche 1,3,4,7 * | 1,3-5 | |
| A | * Seite 44 *<br>-- | 2 | |
| D,A | DD - A - 71 016 (KOCHMANN)<br>* Spalte 1, Zeilen 21-29 *<br>---- | 1,3 | |

RECHERCHIERTE SACHGEBIETE (Int Cl 4)

C 07 D 239/00
C 07 D 417/00
C 07 D 285/00

Der vorliegende Recherchenbericht wurde für alle Patentansprüche erstellt.

| Recherchenort | Abschlußdatum der Recherche | Prüfer |
|---|---|---|
| WIEN | 22-11-1985 | ONDER |

KATEGORIE DER GENANNTEN DOKUMENTEN

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E älteres Patentdokument, das jedoch erst am oder nach dem Anmeldedatum veröffentlicht worden ist
D in der Anmeldung angeführtes Dokument
L aus andern Gründen angeführtes Dokument

& Mitglied der gleichen Patentfamilie, übereinstimmendes Dokument